# EUROPÄISCHE PATENTSCHRIFT

(11) **EP 1 697 043 B1**
(45) Veröffentlichungstag und Bekanntmachung des Hinweises auf die Patenterteilung: **21.10.2009**
(21) Anmeldenummer: 04801200.9
(22) Anmeldetag: 02.12.2004
(51) Int. Cl.: B01J 29/40, C01B 39/36, C07D 487/08

(54) **ZEOLITHISCHES MATERIAL VOM PENTASIL-STRUKTURTYP,SEINE HERSTELLUNG UND SEINE VERWENDUNG**
PENTASIL-STRUCTUER ZEOLITHIC MATERIAL THE PRODUCTION AND USE THEREOF
MATERIAU ZEOLITHIQUE A STRUCTURE DE TYPE PENTASIL, SA PRODUCTION ET SON UTILISATION

(30) Priorität: 02.12.2003 DE 10356184
(43) Veröffentlichungstag der Anmeldung: 06.09.2006
(73) Patentinhaber: BASF SE, 67056 Ludwigshafen (DE)
(72) Erfinder: BOSCH, Marco, 68159 Mannheim (DE); MÜLLER, Ulrich, 67435 Neustadt (DE); FRAUENKRON, Matthias, 67251 Freinsheim (DE)
(74) Vertreter: Isenbruck, Günter
(86) Internationale Anmeldenummer: PCT/EP2004/013733
(87) Internationale Veröffentlichungsnummer: WO 2005/053842

(56) Entgegenhaltungen:
- WO-A-97/03019
- WO-A-03/004499
- VAN GRIEKEN R ET AL: "Anomalous crystallization mechanism in the synthesis of nanocrystalline ZSM-5" MICROPOROUS AND MESOPOROUS MATERIALS, ELSEVIER SCIENCE PUBLISHING, NEW YORK, US, Bd. 39, Nr. 1-2, September 2000 (2000-09), Seiten 135-147, XP004215019 ISSN: 1387-1811
- REDING G ET AL: "Comparing synthesis routes to nano-crystalline zeolite ZSM-5" MICROPOROUS AND MESOPOROUS MATERIALS, ELSEVIER SCIENCE PUBLISHING, NEW YORK, US, Bd. 57, Nr. 1, 2. Januar 2003 (2003-01-02), Seiten 83-92, XP004395614 ISSN: 1387-1811

## Beschreibung

Die vorliegende Erfindung betrifft ein zeolithisches Material vom Pentasil-Strukturtyp, insbesondere vom Strukturtyp ZSM-5, mit einem Alkali- und Erdalkaligehalt von höchstens 150 ppm und einem molaren Verhältnis von Si zu Al im Bereich von 250 bis 1500, wobei mindestens 95 Gew.-% der kugelförmigen Primärpartikel des zeolithischen Materials einen Durchmesser im Bereich von kleiner oder gleich 1 µm aufweisen und mindestens 90 %, bevorzugt mindestens 95 % aller Primärpartikel kugelförmig sind. Ebenso betrifft die vorliegende Erfindung einen Formkörper, der dieses zeolithische Material enthält sowie die Verwendung des zeolithischen Materials an sich oder des Formkörpers als Katalysator. Weiter betrifft die vorliegende Erfindung ein spezielles Verfahren, in dem das zeolithische Material oder der Formkörper, der dieses Material enthält, als Katalysator eingesetzt wird.

In sämtlichen großtechnischen chemischen Prozessen, in denen Katalysatoren eingesetzt werden, ist man bestrebt, die Standzeit der Katalysatoren und damit die Zeit, in der ohne Katalysatortausch und damit ohne Eingriff in den Prozess gearbeitet werden kann, zu erhöhen. Eine Maßnahme hierbei ist, die Teilchengröße der Katalysatoren zu verkleinern. Im Falle eines kristallinen Katalysatormaterials wie beispielsweise eines zeolithischen Katalysators bedeutet dies, dass die Größe der Kristalle, insbesondere der Zeolithkristalle, möglichst klein gehalten werden soll.

Zeolithe sind bekanntermaßen kristalline Alumosilikate mit geordneten Kanal- und Käfigstrukturen, die Mikroporen aufweisen, die vorzugsweise kleiner als ungefähr 0,9 nm sind. Das Netzwerk solcher Zeolithe ist aufgebaut aus SiO₄- und AlO₄-Tetraedern, die über gemeinsame Sauerstoffbrücken verbunden sind. Eine Übersicht der bekannten Strukturen findet sich beispielsweise bei W. M. Meier, D. H. Olson und Ch. Baerlocher, "Atlas of Zeolithe Structure Types", Elsevier, 5. Auflage, Amsterdam 2001. Dabei sind im einzelnen insbesondere die Typen mit röntgenografischer Zuordnung zur ABW-, ACO-, AEI-, AEL-, AEN-, AET-, AFG-, AFI-, AFN-, AFO-, AFR-, AFS-, AFT-, AFX-, AFY-, AHT-, ANA-, APC-, APD-, AST-, ATN-, ATO-, ATS-, ATT-, ATV-, AWO-, AWW-, BEA-, BIK-, BOG-, BPH-, BRE-, CAN-, CAS-, CFI-, CGF-, CGS-, CHA-, CHI-, CLO-, CON-, CZP-, DAC-, DDR-, DFO-, DFT-, DOH-, DON-, EAB-, EDI-, EMT-, EPI-, ERI-, ESV-, EUO-, FAU-, FER-, GIS-, GME-, GOO-, HEU-, IFR-, ISV-, ITE-, JBW-, KFI-, LAU-, LEV-, LIO-, LOS-, LOV-, LTA-, LTL-, LTN-, MAZ-, MEI-, MEL-, MEP-, MER-, MFI-, MFS-, MON-, MOR-, MSO-, MTF-, MTN-, MTT-, MTW-, MWW-, NAT-, NES-, NON-, OFF-, OSI-, PAR-, PAU-, PHI-, RHO-, RON-, RSN-, RTE-, RTH-, RUT-, SAO-, SAT-, SBE-, SBS-, SBT-, SFF-, SGT-, SOD-, STF-, STI-, STT-, TER-, THO-, TON-, TSC-, VET-, VFI-, VNI-, VSV-, WIE-, WEN-, YUG-, ZON-Struktur sowie zu Mischstrukturen aus zwei oder mehr der vorgenannten Strukturen zu nennen.

Je nach Einsatzgebiet des Katalysators muss dieser, neben der Kristallgröße, eine bestimmte Porengröße bzw. Porenverteilung aufweisen. Beispielsweise bei der Herstellung von Triethylendiamin hat sich der Einsatz eines zeolithischen Katalysators vom Strukturtyp ZSM-5 bewährt. Neben den vorstehend beschriebenen Eigenschaften muss der Katalysator auch eine bestimmte chemische Zusammensetzung aufweisen. Im Falle des ZSM-5-Katalysators ist beispielsweise ein bestimmtes molares Verhältnis von Si zu Al im zeolithischen Material vorteilhaft.

Die Verwendung eines Zeolithkatalysators vom Strukturtyp ZSM-5 bei der Herstellung von Triethylendiamin ist beispielsweise in der WO 01/02404 beschrieben, wobei das molare Verhältnis Si zu Al im Bereich von 100 bis 700 und besonders bevorzugt im Bereich von 150 bis 250 liegt. Über die genaue Herstellung des zeolithischen Aktivmaterials wird dabei keine Aussage getroffen.

Die WO 03/004499 beschreibt ein Verfahren zur selektiven Synthese von Triethylendiamin, bei der ein Zeolith-Katalysator, insbesondere vom ZSM-5-Typ, eingesetzt wird, der ein molares Verhältnis von Si zu einem Metall M im Bereich von größer 100, vorzugsweise von größer 200, weiter bevorzugt von größer 300 bis 40.000 und besonders bevorzugt von 400 bis 5000 aufweist. Dabei wird das Metall M, das in der Oxidationsstufe III bzw. IV auftreten kann, ausgewählt aus der Gruppe Al, B, Fe, Co, Ni, V, Mo, Mn, As, Sb, Bi, La, Ga, In, Y, Sc, Cr und Mischungen davon bzw. Ti, Zr, Ge, Hf, Sn und Mischungen davon. Besonders bevorzugt ist hierbei Alumosilikat. Im erfindungsgemäßen Beispiel wird ein Na-Alumosilikat mit einem Verhältnis von Si zu Al von 1.000 eingesetzt. Über Teilchengrößen wird in dieser Schrift nichts ausgesagt.

Die EP 1 215 211 A1 beschreibt ein Verfahren zur Herstellung von Triethylendiamin, bei dem als Katalysator ein Zeolithkatalysator eingesetzt wird, der ein oder mehrere Metalle M in den Oxidationsstufen II, III oder IV als Oxide enthält, wobei im Falle, dass M gleich Al ist, das molare Verhältnis von SiO₂ zu Al₂O₃ größer als 1.400 ist. Neben ZSM-5 sind noch weitere Katalysatoren vom Strukturtyp ZSM-11, ZSM-23, ZSM-53, NU-87, ZSM-35 sowie Mischstrukturen offenbart. Über die Kristallgröße des zeolithischen Materials wird in dieser Schrift nichts gesagt, und das einzige Beispiel betrifft ein zeolithisches Material, das Si und Ti und damit kein Al enthält.

Die Herstellung eines zeolithischen Materials vom ZSM-5-Typ ist in R. Mostowicz und J. M. Berak, Zeolites (1985) 65-72 beschrieben. Die dort offenbarten Kristalle weisen Größen von deutlich größer 1 µm auf. Weiter wird in dieser Schrift die Aussage getroffen, dass das Verhältnis von Si zu Al keinen Einfluss auf die Kristallgröße hat. Explizit beschrieben sind Verhältnisse von Si zu Al von 30 bzw. 90.

In G. Reding, T. Mäurer und B. Kraushaar-Czametzki, Microporous and Mesoporous Materials 57 (2003) 83-92 wird die Herstellung eines nanokristallinen ZSM-5-Materials mit Kristallen eines Durchmessers von kleiner oder gleich 100 nm beschrieben. Dabei liegt allerdings das molare Verhältnis von Si zu Al bei 60. Im Gegensatz zur vorstehend aufgeführten Schrift wird in Reding et al. die Aussage getroffen, dass, je nach Herstellungsverfahren, die Anwesenheit von Aluminium einen erheblichen Einfluss auf die Teilchengrößenverteilung ausübt.

A. E. Persson, B. J. Schoemann, J. Sterte und J.-E. Otterstedt, Zeolites 15 (1995) 611-619 beschreiben die Herstellung eines ZSM-5-Materials mit Teilchengrößen im Bereich von 130 bis 230 nm. Dabei liegt allerdings das molare Verhältnis von Si zu Al im Bereich von ungefähr 50 bis ungefähr 230. Um kleine Teilchen zu erhalten, wird in dieser Schrift ausdrücklich empfohlen, ZSM-5 mit einem niedrigen Verhältnis von SiO₂ zu Al₂O₃ zu synthetisieren. Im Gegensatz zu einer in dieser Schrift zitierten Literaturstelle (C. S. Cundy, B. M. Lowe, D. M. Sinclair, Faraday Discuss. 95 (1993) 235-252), gemäß der die Teilchengröße mit zunehmender Aluminiumkonzentration zunimmt, wurde von Persson et al. der gegengesetzte Effekt gefunden. Gemäß Persson et al. wurde das zeolithische Material unter Verwendung von NaOH und auch NaCl hergestellt. Versuche ohne den Zusatz einer Na-Verbindung führten zu einer abnehmenden Kristallbildungsrate.

P. A. Jacobs, J. A. Martens, Synthesis of High-Silica Aluminosilicate Zeolites, Sudies in Surface Science and Catalysis, Band 33, Elsevier, Amsterdam 1987, beschreiben auf S. 74 dieser Literaturstelle unter anderem, dass bei der Herstellung von ZSM-5 die gleichzeitige Änderung von zwei Parametern (wie beispielsweise dem molaren Verhältnis von Si zu Al, der Salzzugabe, der SiO₂-Quelle, der Alkalinität des Synthesesystems) unerwartete, schwer vorherzusagende Ergebnisse liefert. Dabei werden beispielsweise Kristallstrukturen bei einem großen molaren Verhältnis von Si zu Al von 750 gezeigt, die das Erscheinungsbild von zusammengewachsenen Scheiben aufweisen. Demgemäß werden in P. A. Jacobs et al. im Wesentlichen deutlich kleinere Si zu Al-Verhältnisse beschrieben, die in der Größenordnung von 14 bis 45 liegen.

Angesichts dieses Standes der Technik lag der vorliegenden Erfindung unter anderem die Aufgabe zugrunde, ein zeolithisches Material mit einem großen molaren Verhältnis von Si zu Al bereitzustellen, das gleichzeitig sehr kleine Kristalle und darüber hinaus einen extrem niedrigen Alkali- und Erdalkalimetallgehalt aufweist.

Demgemäß betrifft die vorliegende Erfindung ein zeolithisches Material vom Pentasil-Strukturtyp mit einem Alkali- und Erdalkaligehalt von höchstens 150 ppm und einem molaren Verhältnis von Si zu Al im Bereich von 250 bis 1500, wobei mindestens 90 % der Primärpartikel des zeolithischen Materials kugelförmig sind und mindestens 95 Gew.-% der kugelförmigen Primärpartikel einen Durchmesser im Bereich von kleiner oder gleich 1 µm aufweisen.

Hinsichtlich des Begriffs "Pentasil-Strukturtyp", wie er im Rahmen der vorliegenden Anmeldung verwendet wird, sei auf die oben erwähnte Literatur "W. M. Meier, D. H. Olson und Ch. Baerlocher, "Atlas of Zeolithe Structure Types", Elsevier, 5. Auflage, Amsterdam 2001" verwiesen. Beispiele für Zeolithe vom Pentasil-Strukturtyp sind etwa Zeolithe mit MFI-Struktur, mit MEL-Struktur oder mit einer MFI-MEL-Mischstruktur. Weitere Angaben zur Pentasilstruktur von Zeolithen finden sich beispielsweise auf der Internet-Homepage der international Zeolite Association unter "http://topaz.ethz.ch/IZA-SC/DisordStructures.htm" sowie in der dort unter der Rubrik "Pentasils" angegebenen Literatur: G. T. Kokotailo et. al., Nature 272 (1978) 437; G. T. Kokotailo et. al., Nature 275 (1978) 119; G. Perego, M. Cesari, J. Appl. Cryst 17 (1984) 403.

Der Begriff "Strukturtyp ZSM-5", wie er im Rahmen der vorliegenden Erfindung verwendet wird, bezeichnet ein zeolithisches Material, wie es in W. M. Meier, D. H. Olson und Ch. Baerlocher, "Atlas of Zeolithe Structure Types", Elsevier, 5. Auflage, Amsterdam 2001, S. 184-185, als Zeolith vom Strukturtyp ZSM-5 beschrieben ist.

Gemäß einer bevorzugten Ausführungsform der vorliegenden Erfindung weisen mindestens 96 Gew.-%, weiter bevorzugt mindestens 97 Gew.-%, weiter bevorzugt mindestens 98 Gew.-% und insbesondere mindestens 99 Gew.-% der kugelförmigen Primärpartikel des zeolithischen Materials einen Durchmesser im Bereich von kleiner oder gleich 1 µm auf.

Der Begriff "kugelförmig", wie er im Rahmen der vorliegenden Erfindung verwendet wird, bezeichnet Primärpartikel, die bei Untersuchung über Scanning Electron Microscopy (SEM) bei einer Vergrößerung im Bereich von 0,5·10⁴ bis 2,0·10⁴ im Wesentlichen frei von scharfen Kanten sind. Demgemäß bezeichnet der Begriff "kugelförmig" beispielsweise rein kugelförmige oder deformiert kugelförmig wie beispielsweise elliptische oder quaderförmige Primärteilchen, wobei im Falle der quaderförmigen Primärteilen bei oben erwähnter Untersuchungsmethode im genannten Auflösungsbereich die Kanten abgerundet und nicht scharf sind.

Gemäß einer weiter bevorzugten Ausführungsform der vorliegenden Erfindung sind mindestens 91 %, weiter bevorzugt mindestens 92 %, weiter bevorzugt mindestens 93 %, weiter bevorzugt mindestens 94 %, weiter bevorzugt mindestens 95 %, weiter bevorzugt mindestens 96 % und insbesondere mindestens 97 % der Primärpartikel des zeolithischen Materials kugelförmig.

Der Begriff "Alkali- und Erdalkaligehalt", wie er im Rahmen der vorliegenden Erfindung verwendet wird, bezeichnet den Gehalt des zeolithischen Materials an Alkalimetall, gerechnet als Alkalimetalloxid, und Erdalkalimetall, gerechnet als Erdalkalimetalloxid, wobei unter dem Begriff "Alkali" die Summe aus sämtlichen Alkalimetallen und unter dem Begriff "Erdalkali" die Summe aus sämtlichen Erdalkalimetallen, jeweils gerechnet als die entsprechenden Oxide, zu verstehen ist.

Gemäß einer bevorzugten Ausführungsform der vorliegenden Erfindung beträgt der Alkali- und Erdalkaligehalt des zeolithischen Materials höchstens 125 ppm, weiter bevorzugt höchstens 100 ppm, weiter bevorzugt höchstens 75 ppm und insbesondere bevorzugt höchstens 50 ppm. Im Rahmen der vorliegenden Erfindung sind auch Alkali- und Erdalkaligehalt des zeolithischen Materials von höchstens 25 ppm und oder höchstens 10 ppm möglich.

Demgemäß betrifft die vorliegende Erfindung auch ein zeolithisches Material, wie oben beschrieben, wobei der Alkali- und Erdalkaligehalt höchstens 100 ppm beträgt.

Die Bestimmung des Alkali- und Erdalkaligehalts des zeolithischen Materials erfolgte im Rahmen der vorliegenden Erfindung gemäß nachfolgend beschriebener Methode: 0,1 bis 0,4 g einer Probe des Zeolithmaterials wurden in einer Platinschale eingewogen und mit je 10 ml Schwefelsäure und Flusssäure versetzt. Anschließend wurde auf einer Heizplatte erhitzt und bis zur Trockene abgeraucht. Nach dem Abkühlen wurde der Rückstand mit konzentrierter Salzsäure und etwa dem gleichen Volumen Wasser versetzt und unter Erwärmen gelöst. Die erhaltene Lösung wurde quantitativ in einen 50 ml-Messkolben gespült und mit Wasser bis zur Marke aufgefüllt. In der Regel wurde ein Blindansatz entsprechend hergestellt. Die so hergestellten Lösungen wurden durch Atomabsorption mit Flammentechnik für die zu bestimmenden Elemente analysiert. Im Atomabsorptionsspektrometer wurde als Brenngas C₂H₂ / Luft verwendet.

Was die Primärpartikel des zeolithischen Materials anbelangt, so sind Durchmesser von weniger als 1 µm bevorzugt. Weiter bevorzugt sind Durchmesser von höchstens 900 nm, weiter bevorzugt von höchstens 800 nm, weiter bevorzugt von höchstens 700 nm, weiter bevorzugt von höchstens 600 nm und besonders bevorzugt von höchstens 500 nm. Weiter bevorzugt weisen die Primärpartikel des zeolithischen Materials einen Durchmesser im Bereich von mindestens 10 nm, weiter bevorzugt von mindestens 20 nm, weiter bevorzugt von mindestens 30 nm, weiter bevorzugt von mindestens 40 nm und besonders bevorzugt von mindestens 50 nm auf. Die Durchmesser liegen besonders bevorzugt in einem Bereich von 50 bis 500 nm, weiter besonders bevorzugt von 50 bis 400 nm, weiter besonders bevorzugt von 50 bis 300 nm, weiter besonders bevorzugt von 50 bis 250 nm und ganz besonders bevorzugt von 50 bis 200 nm.

Demgemäß betrifft die vorliegende Erfindung auch ein zeolithisches Material, wie oben beschrieben, wobei die Durchmesser der Primärpartikel des zeolithischen Materials im Bereich von 50 bis 250 nm liegen.

Gemäß einer weiteren Ausführungsform der vorliegenden Erfindung kann der Durchmesser auch im Bereich von 50 bis 100 nm oder im Bereich von 100 bis 150 nm oder im Bereich von 150 bis 200 nm oder im Bereich von 200 bis 250 nm liegen.

Die Durchmesser der Primärpartikel, wie sie im Rahmen der vorliegenden Erfindung beschrieben werden, können beispielsweise über die elektronenmikroskopischen Verfahren SEM (Scanning Electron Microscopy) und TEM (Transmission Electron Microscopy) bestimmt werden. Die im Rahmen der vorliegenden Erfindung beschriebenen Durchmesser wurden über SEM bestimmt.

Was das molare Verhältnis von Si zu Al im zeolithischen Material anbelangt, so liegt dieses bevorzugt im Bereich von 300 zu 1000, weiter bevorzugt im Bereich von 300 bis 900, weiter bevorzugt im Bereich von 300 bis 800, weiter bevorzugt im Bereich von 300 bis 700 und insbesondere bevorzugt im Bereich von 350 bis 600.

Demgemäß betrifft die vorliegende Erfindung auch ein zeolithisches Material, wie oben beschrieben, wobei das molare Verhältnis von Si zu Al im Bereich von 350 bis 600 liegt.

Gemäß weiterer Ausführungsformen der vorliegenden Erfindung können die molaren Verhältnisse von Si zu Al auch im Bereich von 400 bis 600 oder im Bereich von 450 bis 500 liegen.

Das erfindungsgemäße kristalline zeolithische Material vom Pentasil-Strukturtyp, insbesondere vom Strukturtyp ZSM-5, weist bevorzugt eine monodisperse Partikelverteilung auf, wobei der Variationskoeffizient weniger als 50 %, bevorzugt weniger als 25 % und besonders bevorzugt weniger als 10 % beträgt. Der Variationskoeffizient gibt hierbei an, wieviel Prozent vom arithmetischen Mittel die Standardabweichung beträgt. Der Variationskoeffizient ist also gleich 100 * (delta(X)/X), wobei delta(X) für die Standardabweichung und X für das arithmetische Mittel der Partikelgröße steht. Die Partikelgrößenverteilung erfolgt hierbei über Laserbeugungsspektrometrie gemäß DIN 13320.

Die spezifische Oberfläche des erfindungsgemäßen kristallinen zeolithischen Materials, bestimmt gemäß DIN 66131 (BET), liegt im Allgemeinen bei mindestens 350 m²/g, bevorzugt bei mindestens 400 m²/g und insbesondere bevorzugt bei mindestens 425 m²/g. Beispielsweise bevorzugt liegt die spezifische Oberfläche im Bereich von 350 bis 500 m²/g, weiter bevorzugt im Bereich von 400 bis 500 m²/g und insbesondere bevorzugt im Bereich von 425 bis 500 m²/g.

Das Porenvolumen des erfindungsgemäßen kristallinen zeolithischen Materials, bestimmt gemäß DIN 66134 (Langmuir), liegt im Allgemeinen bei mindestens 0,6 ml/g, bevorzugt bei mindestens 0,7 ml/g und insbesondere bevorzugt bei mindestens 0,8 ml/g. Beispielsweise bevorzugt liegt das Porenvolumen im Bereich von 0,6 bis 1,5 ml/g, weiter bevorzugt im Bereich von 0,7 bis 1,4 ml/g und insbesondere bevorzugt im Bereich von 0,8 bis 1,3 ml/g.

Das erfindungsgemäße zeolithische Material kann generell gemäß sämtlicher geeigneter Verfahren hergestellt werden, die zu dem wie oben spezifizierten zeolithischen Material vom Pentasil-Strukturtyp und insbesondere bevorzugt zum ZSM-5-Strukturtyp führen. Zu möglichen Pentasil-Strukturtypen sei auf die oben aufgeführte Liste verwiesen. Beispielhaft seien die Strukturtypen MFI, MEL und die Mischstruktur aus MFI und MEL genannt.

Im Allgemeinen werden die genannten Zeolithe vom Pentasil-Strukturtyp, insbesondere vom Strukturtyp ZSM-5 dadurch hergestellt, dass eine Mischung aus einer SiO₂-Quelle sowie aus einer Al-Quelle und einer stickstoffhaltigen Base als Templat ("Schablonen-Verbindung"), gegebenenfalls noch unter Hinzufügen mindestens einer basischen Verbindung unter Eigendruck oder unter erhöhtem Druck in einem Druckbehälter unter erhöhter Temperatur im Zeitraum von mehreren Stunden bis zu einigen Tagen umgesetzt wird, wobei ein kristallines Produkt entsteht. Dieses wird abgetrennt, gewaschen, getrocknet und zur Entfernung der organischen Stickstoffbase bei erhöhter Temperatur calciniert. In dem so erhaltenen Pulver liegt Al zumindest teilweise innerhalb des Zeolithgitters in wechselndem Anteil mit 4-, 5- oder 6-facher Koordination vor.

Das erfindungsgemäße Verfahren zeichnet sich unter anderem dadurch aus, dass, im Gegensatz zu vielen Verfahren, die im Stand der Technik beschrieben sind, keine Natriumverbindung und auch keine andere Alkalimetallverbindung und/oder Erdalkalimetallverbindung als Starter eingesetzt werden. Damit wird vermieden, dass aus dem zeolithischen Material, das nur die oben beschriebenen, sehr geringen Mengen an Alkalimetall enthalten soll, oder eine seiner Vorstufen Alkalimetall und/oder Erdalkalimetall in mindestens einem zusätzlichen Reaktionsschritt entfernt werden muss. Demgemäß beschreibt die vorliegende Erfindung auch ein wie oben beschriebenes Verfahren, das dadurch gekennzeichnet ist, dass als Startmaterial und/oder Edukt bei der Synthese des mindestens einen zeolithischen Materials keine Alkalimetallverbindung und keine Erdalkalimetallverbindung eingesetzt werden.

Gemäß einer bevorzugten Ausführungsform betrifft die vorliegende Erfindung daher ein Verfahren zur Herstellung eines zeolithischen Materials, wie oben beschrieben, umfassend die Schritte
(i) Herstellung eines Gemischs, enthaltend mindestens eine SiO₂-Quelle, mindestens eine Aluminiumquelle und mindestens eine Templatverbindung, wobei das Gemisch höchstens 150 ppm Alkali- und Erdalkalimetall enthält und wobei die mindestens eine SiO₂-Quelle und die mindestens eine Aluminiumquelle in einem Mengenverhältnis eingesetzt werden, die die Bildung eines kristallinen Materials mit einem molaren Verhältnis von Si zu Al im Bereich von 250 bis 1500 erlaubt;
(ii) Umsetzung der im Gemisch enthaltenden Verbindungen unter Erhalt einer Mutterlauge, enthaltend kristallines, mindestens einen Teil der mindestens einen Templatverbindung enthaltendes Material;
(iii) Abtrennen des kristallinen Materials aus der Mutterlauge;
(iv) Entfernen der mindestens einen Templatverbindung aus dem kristallinen Material.

Der Begriff "Primärpartikel", wie er im Rahmen der vorliegenden Erfindung verwendet wird, bezeichnet Partikel, die ausschließlich durch kovalente Wechselwirkung gebildet sind und in denen keine Van-der-Waals-Wechselwirkungen beim Aufbau eines einzelnen Partikels beteiligt sind. Im Rahmen der vorliegenden Erfindung bezieht sich der Begriff "Primärpartikel" auf Kristalle, die aus der Abtrennung aus der Mutterlauge gemäß obenstehend beschriebenem Schritt (iii) resultieren.

Als SiO₂-Quelle können sämtliche Verbindungen eingesetzt werden, die die Herstellung des erfindungsgemäßen zeolithischen Materials erlauben. Bevorzugt werden beispielsweise Kieselsäure oder ein Kieselsol oder ein Gemisch aus zwei oder mehr verschiedenen Kieselsolen oder ein Tetraalkoxysilan oder ein Gemisch aus zwei oder mehr verschiedenen Tetraalkoxysilanen oder ein Gemisch aus mindestens einem Kieselsol und mindestens einem Tetraalkoxysilan oder ein Gemisch aus Kieselsäure und mindestens einem Kieselsol oder ein Gemisch aus Kieselsäure und mindestens einem Kieselsol und mindestens einem Tetraalkoxysilan eingesetzt.

Ganz allgemein können im Rahmen der vorliegenden Erfindung als SiO₂-Quelle Verbindungen oder Gemische aus Verbindungen der allgemeinen Zusammensetzung

Si(OR)₄₋ₓ(OR')ₓ

mit x = 0, 1, 2, 3 oder 4 eingesetzt werden, wobei R und R' voneinander verschieden sein können und jeweils gleich Wasserstoff, C₁-C₈-Alkyl wie beispielsweise Methyl, Ethyl, n-Propyl, iso-Propyl, n-Butyl, iso-Butyl, tert-Butyl, Pentyl, Hexyl, Heptyl oder Octyl, C₄-C₈-Cycloalkyl wie Cyclobutyl, Cyclopentyl, Cyclohexyl, Cycloheptyl oder Cyclooctyl, Aryl, Alkylaryl oder Arylalkyl sein können, oder wobei R und R' gleich sein können und jeweils gleich Wasserstoff, C₁-C₈-Alkyl wie beispielsweise Methyl, Ethyl, n-Propyl, iso-Propyl, n-Butyl, iso-Butyl, tert-Butyl, Pentyl, Hexyl, Heptyl oder Octyl, C₄-C₈-Cycloalkyl wie Cyclobutyl, Cyclopentyl, Cyclohexyl, Cycloheptyl oder Cyclooctyl, Aryl, Alkylaryl oder Arylalkyl sein können.

Gemäß einer bevorzugten Ausführungsform des erfindungsgemäßen Verfahrens wird als SiO₂-Quelle eine Verbindung der allgemeinen Zusammensetzung

Si(OR)₄

oder der allgemeinen Zusammensetzung

Si(OR)₃(OR')

eingesetzt, wobei R' gleich Wasserstoff und R gleich C₁-C₈-Alkyl wie beispielsweise Methyl, Ethyl, n-Propyl, iso-Propyl, n-Butyl, iso-Butyl, tert-Butyl, Pentyl, Hexyl, Heptyl oder Octyl ist.

Ganz besonders bevorzugt wird als SiO₂-Quelle eine Verbindung der allgemeinen Zusammensetzung

Si(OR)₄

eingesetzt, wobei R gleich C₁-C₈-Alkyl wie beispielsweise Methyl, Ethyl, n-Propyl, iso-Propyl, n-Butyl, iso-Butyl, tert-Butyl, Pentyl, Hexyl, Heptyl oder Octyl, weiter bevorzugt Methyl, Ethyl, n-Propyl, iso-Propyl, n-Butyl, iso-Butyl oder tert-Butyl, weiter bevorzugt Methyl, Ethyl, n-Propyl oder iso-Propyl, weiter bevorzugt Methyl oder Ethyl und insbesondere bevorzugt Ethyl ist.

Als Aluminiumquelle können sämtliche Verbindungen eingesetzt werden, die die Herstellung des erfindungsgemäßen zeolithischen Materials erlauben. Besonders bevorzugt werden im erfindungsgemäßen Verfahren als Aluminiumquelle Alumniumnitrat, Aluminiumsulfat oder Trialkoxyaluminate der Zusammensetzung Al(OR)₃ oder ein Gemisch aus zwei oder mehr dieser Verbindungen eingesetzt. Bezüglich der Trialkoxyaluminate der Zusammensetzung Al(OR)₃ können die Reste R gleich oder verschieden voneinander sein und für C₁-C₈-Alkyl wie beispielsweise Methyl, Ethyl, n-Propyl, iso-Propyl, n-Butyl, iso-Butyl, tert-Butyl, Pentyl, Hexyl, Heptyl oder Octyl, C₄-C₈-Cycloalkyl wie Cyclobutyl, Cyclopentyl, Cyclohexyl, Cycloheptyl oder Cyclooctyl, Aryl, Alkylaryl oder Arylalkyl stehen.

Gemäß einer ganz besonders bevorzugten Ausführungsform des erfindungsgemäßen Verfahrens wird als Aluminiumquelle Aluminiumsulfatoctadecahydrat eingesetzt.

Als Templatverbindung können sämtliche Verbindungen eingesetzt werden, die die Herstellung des erfindungsgemäßen zeolithischen Materials erlauben. Besonders bevorzugt werden im erfindungsgemäßen Verfahren als Templatverbindung Tetraalkylammoniumhydroxide der allgemeinen Zusammensetzung

[NRR'R"R"']⁺OH⁻

eingesetzt, wobei R, R', R" und R"' gleich oder voneinander verschieden sein können und jeweils gleich Wasserstoff, C₁-C₈-Alkyl wie beispielsweise Methyl, Ethyl, n-Propyl, iso-Propyl, n-Butyl, iso-Butyl, tert-Butyl, Pentyl, Hexyl, Heptyl oder Octyl, C₄-C₈-Cycloalkyl wie Cyclobutyl, Cyclopentyl, Cyclohexyl, Cycloheptyl oder Cyclooctyl sein können. Gemäß einer weiter bevorzugten Ausführungsform des erfindungsgemäßen Verfahrens sind R, R', R" und R"', weiter bevorzugt C₁-C₈-Alkyl wie beispielsweise Methyl, Ethyl, n-Propyl, iso-Propyl, n-Butyl, iso-Butyl, tert-Butyl, Pentyl, Hexyl, Heptyl oder Octyl, weiter bevorzugt n-Propyl oder iso-Propyl und insbesondere bevorzugt n-Propyl.

Demgemäß betrifft die vorliegende Erfindung auch ein Verfahren, wie oben beschrieben, wobei als SiO₂-Quelle ein Tetraalkoxysilan, als Aluminiumquelle ein Aluminiumsulfat und als Templatverbindung Tetraalkylammoniumhydroxid eingesetzt werden.

Gemäß einer insbesondere bevorzugten Ausführungsform enthält das Gemisch gemäß (i) zusätzlich Wasser.

Gemäß einer insbesondere bevorzugten Ausführungsform werden im erfindungsgemäßen Verfahren als SiO₂-Quelle Tetraethoxysilan, als Aluminiumquelle Aluminiumsulfatoctadecahydrat und als Templatverbindung Tetrapropylammoniumhydroxid eingesetzt.

Demgemäß beschreibt die vorliegende Erfindung auch ein Verfahren, wie oben beschrieben, das dadurch gekennzeichnet ist, dass gemäß (i) ein Gemisch, enthaltend Tetraethoxysilan, Aluminiumsulfatoctadecahydrat, Tetrapropylammoniumhydroxid und Wasser, hergestellt wird.

Die oben genannten, im Gemisch gemäß (i) enthaltenen Verbindungen werden in molaren Verhältnissen eingesetzt, die die Herstellung des erfindungsgemäßen zeolithischen Materials, insbesondere mit dem obenstehend beschriebenen molaren Verhältnis von Si zu Al im Bereich von 250 bis 1500 erlauben. Bevorzugt wird ein Synthesegel als Gemisch gemäß (i) eingesetzt, das folgende molare Zusammensetzung aufweist, wobei Si in der mindestens einen SiO₂-Quelle als SiO₂ gerechnet wird, Al in der mindestens einen Aluminiumquelle als Al₂O₃ gerechnet wird und die Abkürzung TMP für die Templatverbindung steht

x TMP•SiO₂•y Al₂O₃•z H₂O

und x = 0,2 bis 1,0, y = 2,5•10⁻⁴ bis 25•10⁻⁴ und z = 15 bis 100 sind.

Im Allgemeinen weist das Synthesegel, das Gemisch gemäß (i), einen Alkali- und Erdalkaligehalt von höchstens 100 ppm, bevorzugt von höchstens 50 ppm, weiter bevorzugt von höchstens 25 ppm und insbesondere bevorzugt von höchstens 10 ppm auf.

Die Zugabereihenfolge der Bestandteile des Synthesegels ist in der Regel unkritisch. Gemäß einer bevorzugten Ausführungsform des erfindungsgemäßen Verfahrens wird eine Lösung oder eine Suspension, bevorzugt eine Lösung der mindestens einen Templatverbindung vorgelegt und dieser Lösung die mindestens eine SiO₂-Quelle zugegeben. Diese Zugabe findet bevorzugt bei Temperaturen im Bereich von 5 bis 40 °C, weitere bevorzugt im Bereich von 15 bis 35 °C und besonders bevorzugt im Bereich von 20 bis 30 °C statt.

Je nach SiO₂-Quelle und/oder Al-Quelle ist es möglich, dass im Gemisch gemäß (i) durch beispielsweise Hydrolyse ein oder mehrere Alkohole entstehen. Gemäß einer besonders bevorzugten Ausführungsform wird dieser mindestens eine Alkohol vor der Kristallisation des zeolithischen Materials abgetrennt. Dabei können sämtliche geeigneten Abtrennverfahren eingesetzt werden, wobei das Abdestillieren besonders bevorzugt ist. Diese Destillation kann hierbei beispielsweise bevorzugt bei Normaldruck oder unter gegenüber Normaldruck verringertem Druck erfolgen. Weiter bevorzugt wird das Abdestillieren des mindestens einen Alkohols bei Sumpftemperaturen im Bereich von 85 bis 95°C und insbesondere im Bereich von 90 bis 95 °C durchgeführt.

Demgemäß betrifft die vorliegende Erfindung auch ein Verfahren, wie oben beschrieben, wobei vor der Umsetzung gemäß (ii) der im Gemisch gemäß (i) entstehende Alkohol abdestilliert wird.

Im Rahmen des erfindungsgemäßen Verfahrens ist es möglich, die mindestens eine Aluminiumquelle vor oder nach der Abtrennung des mindestens einen Alkohols dem Reaktionsgemisch zuzugeben. Gemäß einer bevorzugten Ausführungsform erfolgt die Zugabe der mindestens einen Aluminiumquelle nach der Abtrennung des mindestens einen Alkohols.

Gemäß einer weiter bevorzugten Ausführungsform des erfindungsgemäßen Verfahrens wird das nach Abtrennen des mindestens einen aus der Hydrolyse erhaltenen Alkohols erhaltene Gemisch, weiter bevorzugt der aus der Destillation erhaltene Sumpf, mit insbesondere bevorzugt Wasser, weiter bevorzugt mit VE-Wasser versetzt. Dabei wird bevorzugt so viel Wasser zugegeben, dass der Destillationsverlust in etwa kompensiert wird.

Die Umsetzung gemäß (ii), aus der kristallines zeolithisches Material in seiner Mutterlauge erhalten wird, findet bevorzugt bei Temperaturen im Bereich von 150 bis 180 °C, besonders bevorzugt im Bereich von 160 bis 180 °C und insbesondere im Bereich von 170 bis 180°C statt.

Gemäß einer insbesondere bevorzugten Ausführungsform des erfindungsgemäßen Verfahrens findet die Umsetzung gemäß (ii) in einem Autoklaven wie beispielsweise in einem Stahlautoklaven statt. Weiter bevorzugt wird zumindest während eines Teils der Umsetzungsdauer das Reaktionsgemisch gerührt.

Die Dauer der Umsetzung gemäß (i) liegt bevorzugt im Bereich von 1 bis 48 h, weiter bevorzugt im Bereich von 4 bis 36 h und besonders bevorzugt im Bereich von 12 bis 24 h.

Der Druck bei dieser Umsetzung gemäß (i) liegt bevorzugt im Bereich von Normaldruck bis 50 bar, weiter bevorzugt im Bereich von 5 bis 25 bar und besonders bevorzugt im Bereich von 10 bis 15 bar.

Demgemäß betrifft die vorliegende Erfindung auch ein Verfahren, wie oben beschrieben, wobei die Umsetzung gemäß (ii) bei einer Temperatur im Bereich von 150 bis 180 °C in einem Autoklaven bei einer Umsetzungsdauer von 1 bis 48 h umgesetzt wird.

Gemäß einer bevorzugten Ausführungsform wird der pH-Wert der Mutterlauge, enthaltend das kristalline zeolithische Material, bevorzugt nach Abkühlen auf in etwa Raumtemperatur und vor dem Abtrennen gemäß (iii) durch Zugabe mindestens einer geeigneten Verbindung auf einen Wert im Bereich von 5,5 bis 7,5 und bevorzugt im Bereich von 6 bis 7 gebracht.

Besonders bevorzugt sind hierbei als geeignete Verbindungen Broenstedt-Säuren wie beispielsweise Salzsäure, Schwefelsäure, Phosphorsäure, Salpetersäure oder Carbonsäuren, Dicarbonsäuren oder Oligo- oder Polycarbonsäuren, die allein oder als Gemisch aus zwei oder mehr davon eingesetzt werden können. Weiter können die genannten Säuren konzentriert oder in verdünnter Lösung eingesetzt werden. Werden die Säuren in Lösung eingesetzt, so ist als Lösungsmittel beispielsweise Wasser besonders bevorzugt. Ganz besonders sind hierbei Säuren, die in einem nachfolgenden Calcinierschritt entfernt werden können wie beispielsweise Carbonsäuren oder Salpetersäure.

Gemäß einer besonders bevorzugten Ausführungsform des erfindungsgemäßen Verfahrens wird das kristalline zeolithische Material vom Pentasil-Strukturtyp, bevorzugt vom Strukturtyp ZSM-5, durch ein geeignetes Verfahren gemäß (iii) aus seiner Mutterlauge abgetrennt und weiter bevorzugt gemäß eines oder mehrerer geeigneter Verfahren getrocknet und wiederum bevorzugt anschließend calciniert. Die Calcinierung kann beispielsweise bevorzugt in einer geeigneten Gasatmosphäre erfolgen, wobei besonders bevorzugt Luft und/oder Magerluft als Gasatmosphäre verwendet wird.

Sämtliche Verfahren zur Abtrennung des kristallinen zeolithischen Materials aus seiner Mutterlauge sind denkbar. Unter anderem seien etwa Filtrations-, Ultrafiltrations-, Diafiltrations-, Zentrifugierverfahren oder etwa Sprühtrocknungs- und Sprühgranulierverfahren genannt. Bevorzugt wird das kristalline zeolithische Material durch Ultrafiltration aus der Mutterlauge abgetrennt. Gemäß einer weiter bevorzugten Ausführungssform des erfindungsgemäßen Verfahren wird das kristalline zeolithische Material durch Filtration aus der Mutterlauge abgetrennt.

Demgemäß beschreibt die vorliegende Erfindung auch ein Verfahren, wie oben beschrieben, wobei das zeolithische Material mittels Ultrafiltration gemäß (iii) aus der Mutterlauge abgetrennt wird.

Demgemäß beschreibt die vorliegende Erfindung weiter auch ein Verfahren, wie oben beschrieben, wobei das zeolithische Material mittels Filtration gemäß (iii) aus der Mutterlauge abgetrennt wird, wobei insbesondere die wie oben beschriebene Einstellung des pH-Werts auf einen Wert im Bereich von 5,5 bis 7,5 und bevorzugt im Bereich von 6 bis 7 erfolgt.

Vor der Abtrennung des kristallinen zeolithischen Materials aus der Mutterlauge ist es möglich, den Gehalt der Mutterlauge an zeolithischem Material durch Aufkonzentrieren zu erhöhen. Details zur Abtrennung des kristallinen zeolithischen Materials aus der Mutterlauge sind auch in der DE-A 102 32 406.9 zu finden, die diesbezüglich durch Bezugnahme in den Kontext der vorliegenden Anmeldung vollumfänglich aufgenommen wird. Beispielsweise kann die gemäß einer Ausführungsform des erfindungsgemäßen Verfahrens für die Ultrafiltration verwendete Membran Trennschichten mit Porendurchmessern von 10 bis 500 nm enthalten, wobei die Geometrie der mindestens einen Membran beispielsweise ausgewählt wird aus der Gruppe bestehend aus Flach-, Rohr-, Multikanalelement-, Kapillar und Wickelgeometrie. Der transmembrane Druck beträgt bei der Ultrafiltration bspw. 0,5 bis 20 bar. Die Temperatur, bei der die Ultrafiltration durchgeführt wird, liegt beispielsweise bevorzugt im Bereich von Raumtemperatur bis 80°C, weiter bevorzugt im Bereich von 30 bis 80 °C.

Das von der Mutterlauge abgetrennte zeolithische Material wird bevorzugt bei Temperaturen im Bereich von im allgemeinen 80 bis 160 °C, bevorzugt von 90 bis 145°C und besonders bevorzugt von 100 bis 130°C getrocknet, wobei die Trocknungsdauer im Allgemeinen bei 6 h oder mehr, beispielsweise im Bereich von 6 bis 24, liegt. Es sind jedoch in Abhängigkeit vom Feuchtigkeitsgehalt des zu trocknenden Materials auch kürzere Trockenzeiten wie beispielsweise ungefähr 1, 2, 3, 4, oder 5 h möglich.

Die anschließend bevorzugt mindestens einmal erfolgende Calcinierung, bei der gemäß (iv) die mindestens eine Templatverbindung und gegebenenfalls die mindestens eine, obenstehend beschrieben Säure aus dem kristallinen Material entfernt werden, wird bei Temperaturen im Bereich von im allgemeinen 400 bis 750 °C, bevorzugt von 450 bis 600 °C und besonders bevorzugt von 490 bis 530°C durchgeführt.

Die Calcinierung kann unter jeder geeigneten Gasatmosphäre erfolgen, wobei Luft und/oder Magerluft bevorzugt sind. Weiter wird die Calcinierung bevorzugt in einem Muffelofen, einem Drehrohrofen und/oder einem Bandkalzinierofen durchgeführt, wobei die Calcinierungsdauer im Allgemeinen bei 1 h oder mehr, beispielsweise im Bereich von 1 bis 24 oder im Bereich von 4 bis 12 liegt. Demgemäß ist es im Rahmen erfindungsgemäßen Verfahrens beispielsweise möglich, das zeolithische Material einmal, zweimal oder öfter für jeweils mindestens 1 h wie beispielsweise jeweils im Bereich von 4 bis 12 h, bevorzugt im Bereich von 4 bis 8 h zu calcinieren, wobei die Temperaturen während eines Calcinierungsschrittes gleich bleiben oder kontinuierlich oder diskontinuierlich geändert werden können. Wird zweimal oder öfter calciniert, können sich die Calcinierungstemperaturen in den einzelnen Schritten unterscheiden oder gleich sein.

Demgemäß betrifft die vorliegende Erfindung auch ein Verfahren, wie oben beschrieben, wobei das gemäß (iii) abgetrennte kristalline Material zunächst bei einer Temperatur im Bereich von 80 bis 160 °C getrocknet und anschließend gemäß (iv) bei einer Temperatur im Bereich von 400 bis 750°C calciniert wird.

Gemäß weiterer Ausführungsformen des erfindungsgemäßen Verfahrens kann der Zeolith nach der Abtrennung und/oder nach dem Trocknen und/oder nach dem Calcinieren mindestens einem Waschprozess unterworfen werden, wobei das abgetrennte kristalline Material mit mindestens einer geeigneten Waschsubstanz in Kontakt gebracht wird. Ganz besonders bevorzugt wird das kristalline zeolithische Material vor dem Trocknen mit Wasser gewaschen. Der Waschprozess kann im Rahmen der vorliegenden Erfindung sowohl mit Wasser in flüssigem Zustand als auch mit Wasserdampf erfolgen, wobei auch Ausführungsformen möglich sind, bei denen der Waschprozess mit sowohl Wasser in flüssigem Zustand und Wasserdampf, gleichzeitig oder in beliebiger Reihenfolge, durchgeführt wird. Bevorzugt wird mit flüssigem Wasser gewaschen.

Wird die Behandlung mit Wasserdampf gewählt, so wird das abgetrennte zeolithische Material besonders bevorzugt mit Wasserdampf bei Temperaturen im Bereich von 100 bis 750 °C, bevorzugt von 100 bis 250 °C und besonders bevorzugt von 120 bis 175 °C in Kontakt gebracht, wobei dieses Inkontaktbringen bevorzugt zwischen 12 und 48 h andauert.

Ganz besonders bevorzugt findet dieses Inkontaktbringen in einem Autoklaven statt.

Demgemäß beschreibt die vorliegende Erfindung auch ein Verfahren, wie oben beschrieben, wobei das zeolithische Material nach Schritt (iv) in einem Autoklaven mit Wasser in Kontakt gebracht wird.

Zusätzlich oder statt des mindestens einen Waschprozesses kann das abgetrennte zeolithische Material mit einer konzentrierten oder verdünnten Broenstedt-Säure oder einem Gemisch aus zwei oder mehr Broenstedt-Säuren behandelt werden. Geeignete Säuren sind beispielsweise Salzsäure, Schwefelsäure, Phosphorsäure, Salpetersäure oder Carbonsäuren, Dicarbonsäuren oder Oligo- oder Polycarbonsäuren wie beispielsweise Nitrilotriessigsäure, Sulfosalicylsäure oder Ethylendiaminotetraessigsäure. Gemäß einer bevorzugten Ausführungsform des erfindungsgemäßen Verfahrens wird auf diesen Schritt der Behandlung des abgetrennten zeolithischen Materials mit einer konzentrierten oder verdünnten Broenstedt-Säure oder einem Gemisch aus zwei oder mehr Broenstedt-Säuren verzichtet.

Im Falle, dass der Zeolith nach dem Abtrennen aus der Mutterlauge wie oben beschrieben getrocknet und/oder calciniert wurde und nach dem Trocknen und/oder dem Calcinieren einem Waschprozess und/oder einer Behandlung mit mindestens einer Broenstedtsäure unterworfen wurde, schließt sich gemäß einer besonders bevorzugten Ausführungsform der vorliegenden Erfindung ein nochmaliges Trocknen und/oder Calcinieren an.

Diese Trocknung erfolgt dabei bei Temperaturen im Bereich von im allgemeinen 80 bis 160°C, bevorzugt von 90 bis 145 °C und besonders bevorzugt von 100 bis 130 °C. Die anschließend bevorzugt erfolgende Calcinierung wird bei Temperaturen im Bereich von im allgemeinen 400 bis 750 °C, bevorzugt von 450 bis 600 °C und besonders bevorzugt von 490 bis 530 °C durchgeführt.

Demgemäß betrifft die vorliegende Erfindung auch ein Verfahren, wie oben beschrieben, wobei das zeolithische Material nach Schritt (iv) in einem Autoklaven mit Wasser in Kontakt gebracht wird und anschließend bei einer Temperatur im Bereich von 80 bis 160 °C getrocknet und anschließend bei einer Temperatur im Bereich von 400 bis 750 °C calciniert wird.

Ebenso betrifft die vorliegende Erfindung ein zeolithisches Material vom Pentasiltyp mit einem Alkali- und Erdalkaligehalt von höchstens 150 ppm und einem molaren Verhältnis von Si zu Al im Bereich von 250 bis 1500, wobei mindestens 90 % der Primärpartikel des zeolithischen Materials kugelförmig sind und mindestens 95 Gew.-% der kugelförmigen Primärpartikel einen Durchmesser im Bereich von kleiner oder gleich 1 µm aufweisen, das erhältlich ist durch ein Verfahren gemäß einer der oben beschriebenen Ausführungsformen.

Das erfindungsgemäße zeolithische Material oder/und das derart erfindungsgemäß hergestellte zeolithische Material kann generell in sämtlichen Verfahren oder Arbeitsschritten eingesetzt werden, in denen die Eigenschaften eines zeolithischen Materials erwünscht sind. Ganz besonders bevorzugt wird das erfindungsgemäße oder erfindungsgemäß hergestellte zeolithische Material als Katalysator bei chemischen Umsetzungen eingesetzt.

Gerade im Bereich der Katalysatoren ist es von Anwenderseite her oft erwünscht, nicht das kristalline, katalytisch aktive Material an sich einzusetzen, sondern das Material, das in Formkörpern vorliegt. Diese Formkörper sind gerade in vielen großindustriellen Verfahren nötig, um beispielsweise chemische Umsetzungen in beispielsweise Rohrreaktoren oder Rohrbündelreaktoren in unter anderem Festbettfahrweise sinnvoll betreiben zu können.

Demgemäß betrifft die vorliegende Erfindung auch einen Formkörper, enthaltend ein wie oben beschriebenes zeolithisches Material.

Generell kann der Formkörper neben dem erfindungsgemäßen zeolithischen Material sämtliche denkbaren weiteren Verbindungen umfassen, solange gewährleistet ist, dass der resultierende Formkörper für die erwünschte Anwendung geeignet ist.

Im Rahmen der vorliegenden Erfindung ist es bevorzugt, dass bei der Herstellung des Formkörpers mindestens ein geeignetes Bindermaterial verwendet wird. Im Rahmen dieser bevorzugten Ausführungsform wird weiter bevorzugt ein Gemisch aus zeolithischem Material und dem mindestens einen Bindermaterial hergestellt.

Demgemäß beschreibt die vorliegende Erfindung auch ein Verfahren zur Herstellung eines Formkörpers, enthaltend ein wie oben beschriebenes zeolithisches Material, umfassend den Schritt
(I) Herstellen eines Gemischs, enthaltend ein wie oben beschriebenes zeolithisches Material oder ein zeolithisches Material, erhältlich gemäß einem wie oben beschriebenen Verfahren, und mindestens ein Bindermaterial.

Als Bindermaterial sind generell sämtliche Verbindungen geeignet, die eine über die ohne Bindemittel gegebenenfalls vorhandene Physisorption hinausreichende Adhäsion und/oder Kohäsion zwischen den zu bindenden Teilchen des zeolithischen Materials vermittelt. Beispiele für solche Bindermaterialien sind etwa Metalloxide wie beispielsweise SiO₂, Al₂O₃, TiO₂, ZrO₂ oder MgO oder Tone oder Gemische aus zwei oder mehr dieser Verbindungen.

Als Al₂O₃-Bindemittel sind insbesondere Tonmineralien und natürlich vorkommende oder künstlich hergestellte Aluminiumoxide wie beispielsweise alpha-, beta-, gamma-, delta-, eta-, kappa-, chi- oder theta-Aluminiumoxid sowie deren anorganische oder metallorganische Vorläuferverbindungen wie beispielsweise Gibbsit, Bayerit, Boehmit, Pseudoboehmit oder Trialkoxyaluminate wie beispielsweise Aluminiumtriisopropylat geeignet. Weitere bevorzugte Bindemittel sind amphiphile Verbindungen mit einem polaren und einem nichtpolaren Anteil sowie Graphit. Weitere Bindemittel sind etwa Tone wie etwa Montmorillonite, Kaoline, Bentonite, Halloysite, Dickite, Nacrite oder Anaxite.

Diese Bindemittel können als solche eingesetzt werden. Ebenso ist es im Rahmen der vorliegenden Erfindung auch möglich, Verbindungen einzusetzen, aus denen in mindestens einem weiteren Schritt bei der Herstellung der Formkörper das Bindemittel gebildet wird. Beispiele für solche Bindemittelvorstufen sind etwa Tetraalkoxysilane, Tetraalkoxytitanate, Tetraalkoxyzirkonate oder ein Gemisch aus zwei oder mehr verschiedenen Tetraalkoxysilanen oder ein Gemisch aus zwei oder mehr verschiedenen Tetraalkoxytitanaten oder ein Gemisch aus zwei oder mehr verschiedenen Tetraalkoxyzirkonaten oder ein Gemisch aus mindestens einem Tetraalkoxysilan und mindestens einem Tetraalkoxytitanat oder aus mindestens einem Tetraalkoxysilan und mindestens einem Tetraalkoxyzirkonat oder aus mindestens einem Tetraalkoxytitanat und mindestens einem Tetraalkoxyzirkonat oder ein Gemisch aus mindestens einem Tetraalkoxysilan und mindestens einem Tetraalkoxytitanat und mindestens einem Tetraalkoxyzirkonat.

Ganz besonders bevorzugt sind im Rahmen der vorliegenden Erfindung Bindemittel, die entweder ganz oder teilweise aus SiO₂ bestehen oder eine Vorstufe zu SiO₂ darstellen, aus der in mindestens einem weiteren Schritt bei der Herstellung der Formkörper SiO₂ gebildet wird. In diesem Zusammenhang können sowohl kolloidales Siliciumdioxid als auch so genanntes "wet process"-Siliciumdioxid als auch so genanntes "dry process"-Siliciumdioxid eingesetzt werden. In diesen Fällen handelt es sich ganz besonders bevorzugt um amorphes Siliciumdioxid, wobei die Größe der Siliciumdioxidpartikel im Bereich von 5 bis 100 nm liegt und die Oberfläche der Siliciumdioxidpartikel im Bereich von 50 bis 500 m²/g liegt.

Kolloidales Siliciumdioxid, bevorzugt als alkalische und/oder ammoniakalische Lösung, weiter bevorzugt als ammoniakalische Lösung, ist unter anderem etwa kommerziell erhältlich als Ludox®, Syton®, Nalco® oder Snowtex®.

"Wet process"-Siliciumdioxid ist unter anderem etwa kommerziell erhältlich als Hi-Sil®, Ultrasil®, Vulcasil®, Santocel®, Valron-Estersil®, Tokusil® oder Nipsil®.

"Dry process"-Siliciumdioxid ist unter anderem etwa kommerziell erhältlich als Aerosil®, Reolosil®, Cab-O-Sil®, Fransil® oder ArcSilica®.

Unter anderem bevorzugt wird im Rahmen der vorliegenden Erfindung eine ammoniakalische Lösung von kolloidalem Siliciumdioxid.

Demgemäß betrifft die vorliegende Erfindung auch einen Formkörper, wie oben beschrieben, zusätzlich enthaltend SiO₂ als Bindermaterial.

Ebenso betrifft die vorliegende Erfindung auch ein Verfahren, wie oben beschrieben, wobei das gemäß (I) eingesetzte Bindermaterial ein SiO₂ enthaltendes oder bildendes Bindermaterial ist.

Demgemäß betrifft die vorliegende Erfindung auch ein Verfahren, wie oben beschrieben, wobei das Bindemittel ein kolloidales Siliciumdioxid ist.

Bevorzugt werden die Bindemittel in einer Menge eingesetzt, die zu letztlich resultierenden Formkörpern führt, deren Bindemittelgehalt im Bereich von bis zu 80 Gew.-% liegen, weiter bevorzugt im Bereich von 5 bis 80 Gew.-%, weiter bevorzugt im Bereich von 10 bis 70 Gew.-%, weiter bevorzugt im Bereich von 10 bis 60 Gew.-%, weiter bevorzugt im Bereich von 15 bis 50 Gew.-%, weiter bevorzugt im Bereich von 15 bis 45 Gew.-% und besonders bevorzugt im Bereich von 15 bis 40 Gew.-%, jeweils bezogen auf das Gesamtgewicht des letztlich resultierenden Formkörpers.

Das Gemisch aus Bindemittel oder Vorstufe zu einem Bindemittel und dem zeolithischen Material kann zur weiteren Verarbeitung und zur Ausbildung einer plastischen Masse mit mindestens einer weiteren Verbindung versetzt werden. Unter anderem bevorzugt sind hier Porenbildner zu nennen.

Als Porenbildner können im erfindungsgemäßen Verfahren sämtliche Verbindungen eingesetzt werden, die bezüglich des fertigen Formkörpers eine bestimmte Porengröße, eine bestimmte Porengrößenverteilung und/oder bestimmte Porenvolumina bereitstellen.

Bevorzugt werden als Porenbildner im erfindungsgemäßen Verfahren Polymere eingesetzt, die in Wasser oder in wässrigen Lösungsmittelgemischen dispergier-, suspendier- oder emulgierbar sind. Bevorzugte Polymere sind hierbei polymere Vinylverbindungen wie beispielsweise Polyalkylenoxide wie Polyethylenoxide, Polystyrol, Polyacrylate, Polymethacrylate, Polyolefine, Polyamide und Polyester, Kohlenhydrate wie etwa Cellulose oder Cellulosederivate, wie beispielsweise Methylcellulose, oder Zucker oder Naturfasern. Weitere geeignete Porenbildner sind etwa Pulp oder Graphit.

Werden bei der Herstellung des Gemischs gemäß (i) Porenbildner eingesetzt, so liegt der Gehalt des Gemischs gemäß (i) an Polymer bevorzugt im Bereich von 5 bis 90 Gew.-%, weiter bevorzugt im Bereich von 15 bis 75 Gew.-% und besonders bevorzugt im Bereich von 25 bis 55 Gew.-%, jeweils bezogen auf die Menge an zeolithischem Material im Gemisch gemäß (i).

Sollte dies für die zu erzielende Porengrößenverteilung erwünscht sein, kann auch ein Gemisch aus zwei oder mehr Porenbildnern eingesetzt werden.

Die Porenbildner werden in einer besonders bevorzugten Ausführungsform des erfindungsgemäßen Verfahrens, wie untenstehend beschrieben, in einem Schritt (V) durch Calcinieren unter Erhalt des porösen Formkörpers entfernt. Gemäß einer bevorzugten Ausführungsform des erfindungsgemäßen Verfahrens werden dabei Formkörper erhalten, die Poren, bestimmt gemäß DIN 66134, im Bereich von mindestens 0,6 ml/g, bevorzugt im Bereich von 0,6 bis 0,8 ml/g und insbesondere bevorzugt im Bereich von mehr als 0,6 ml/g bis 0,8 ml/g aufweisen.

Die spezifische Oberfläche des erfindungsgemäßen Formkörpers, bestimmt gemäß DIN 66131, liegt im Allgemeinen bei mindestens 350 m²/g, bevorzugt bei mindestens 400 m²/g und insbesondere bevorzugt bei mindestens 425 m²/g. Beispielsweise kann die spezifische Oberfläche im Bereich von 350 bis 500 m²/g oder 400 bis 500 m²/g oder 425 bis 500 m²/g liegen.

Demgemäß betrifft die vorliegende Erfindung auch einen Formkörper, wie oben beschrieben, mit einer spezifischen Oberfläche von mindestens 350 m²/g, enthaltend Poren mit einem Porenvolumen von mindestens 0,6 ml/g.

Bei der Herstellung des Gemisches gemäß (I) wird im Rahmen einer ebenfalls bevorzugten Ausführungsform des erfindungsgemäßen Verfahrens mindestens ein Anteigungsmittel zugegeben.

Als Anteigungsmittel können alle dafür geeigneten Verbindungen verwendet werden. Vorzugsweise sind dies organische, insbesondere hydrophile Polymere wie beispielsweise Cellulose, Cellulosederivate wie beispielsweise Methylcellulose, Stärke wie beispielsweise Kartoffelstärke, Tapetenpflaster, Polyacrylate, Polymethacrylate, Polyvinylalkohol, Polyvinylpyrrolidon, Polyisobuten oder Polytetrahydrofuran.

Insbesondere können demgemäß als Anteigungsmittel Verbindungen eingesetzt werden, die auch als Porenbildner wirken.

Diese Anteigungsmittel werden in einer besonders bevorzugten Ausführungsform des erfindungsgemäßen Verfahrens, wie untenstehend beschrieben, in einem Schritt (V) durch Calcinieren unter Erhalt des porösen Formkörpers entfernt.

Gemäß einer weiteren Ausführungsform der vorliegenden Erfindung wird bei der Herstellung des Gemisches gemäß (I) mindestens ein saurer Zusatzstoff zugesetzt. Ganz besonders bevorzugt sind organische saure Verbindungen, die sich in dem bevorzugten Schritt (V), wie untenstehend beschrieben, durch Calcinieren entfernen lassen. Besonders bevorzugt sind Carbonsäuren wie beispielsweise Ameisensäure, Oxalsäure und/oder Citronensäure. Ebenso ist es möglich, zwei oder mehr dieser saueren Verbindungen einzusetzen.

Die Zugabereihenfolge der Bestandteile des das zeolithische Material enthaltenden Gemischs gemäß (I) ist nicht kritisch. Es ist sowohl möglich, zuerst das mindestens eine Bindemittel zuzugeben, anschließend den mindestens einen Porenbildner, die mindestens eine saure Verbindung und zum Schluss das mindestens eine Anteigungsmittel, als auch die Reihenfolge bezüglich des mindestens einen Bindemittels, des mindestens einen Porenbildners, der mindestens einen sauren Verbindung und des mindestens einen Anteigungsmittels zu vertauschen.

Nach der Zugabe des Bindemittels zum zeolithhaltigen Feststoff, dem gegebenenfalls mindestens eine der oben beschriebenen Verbindungen bereits zugegeben worden war, wird das Gemisch gemäß (I) in der Regel 10 bis 180 Minuten homogenisiert. Besonders bevorzugt werden zur Homogenisierung unter anderem Kneter, Koller oder Extruder eingesetzt. Bevorzugt wird das Gemisch geknetet. Im industriellen Maßstab wird zur Homogenisierung bevorzugt gekollert.

Demgemäß beschreibt die vorliegende Erfindung auch ein Verfahren, wie oben beschrieben, umfassend die Schritte
(I) Herstellen eines Gemischs, enthaltend ein wie oben beschriebenes zeolithisches Material oder ein zeolithisches Material, erhältlich gemäß einem wie oben beschriebenen Verfahren, und mindestens ein Bindermaterial;
(II) Kneten des Gemischs.

Bei der Homogenisierung wird in der Regel bei Temperaturen im Bereich von ungefähr 10°C bis zum Siedepunkt des Anteigungsmittels und Normaldruck oder leichtem überatmosphärischem Druck gearbeitet. Danach kann gegebenenfalls mindestens eine der oben beschriebenen Verbindungen zugegeben werden. Das so erhaltene Gemisch wird solange homogenisiert, vorzugsweise geknetet, bis eine verstrangbare plastische Masse entstanden ist.

Das homogenisierte Gemisch wird gemäß einer weiter bevorzugten Ausführungsform der vorliegenden Erfindung verformt.

Im Rahmen der vorliegenden Erfindung sind für die Verfahren im Rahmen der Formgebung solche Verfahren bevorzugt, bei denen die Verformung durch Extrusion in üblichen Extrudern, beispielsweise zu Strängen mit einem Durchmesser von bevorzugt 1 bis 10 mm und besonders bevorzugt 2 bis 5 mm, erfolgt. Derartige Extrusionsvorrichtungen werden beispielsweise in Ullmann's Enzyklopädie der Technischen Chemie, 4. Auflage, Bd. 2, S. 295 ff., 1972 beschrieben. Neben der Verwendung eines Extruders wird ebenfalls vorzugsweise eine Strangpresse zur Verformung verwendet.

Prinzipiell können jedoch zur Formgebung alle bekannten und/oder geeigneten Knet- und Verformungsvorrichtungen bzw. Verfahren eingesetzt werden. Unter anderem sind hierbei zu nennen:
(i) Brikettieren, d.h. mechanisches Verpressen mit oder ohne Zusatz von zusätzlichem Bindermaterial;
(ii) Pelletieren, d.h. Kompaktieren durch kreisförmige und/oder rotierende Bewegungen;
(iii) Sintern, d.h. das zu verformende Materials wird einer thermischen Behandlung ausgesetzt.

Beispielsweise kann die Formgebung aus der folgenden Gruppe ausgewählt sein, wobei die Kombination von mindestens zwei dieser Methoden explizit eingeschlossen ist: Brikettieren durch Stempelpressen, Walzenpressen, Ringwalzenpressen, Brikettieren ohne Bindemittel Pelletieren, Schmelzen, Spinning-Techniken, Abscheidung, Schäumen, Sprühtrocknen; Brennen im Schachtofen, Konvektionsofen, Wanderrost, Drehrohrofen, Kollern.

Das Kompaktieren kann bei Umgebungsdruck oder bei gegenüber dem Umgebungsdruck erhöhtem Druck stattfinden, beispielsweise in einem Druckbereich von 1 bar bis zu mehreren hundert bar. Weiterhin kann das Kompaktieren bei Umgebungstemperatur oder bei gegenüber der Umgebungstemperatur erhöhter Temperatur stattfinden, beispielsweise in einem Temperaturbereich von 20 bis 300 °C. Ist Trocknen und/oder Brennen Bestandteil des Formgebungsschritt, so sind Temperaturen bis zu 1500 °C denkbar. Schließlich kann das Kompaktieren in der Umgebungs-Atmosphäre stattfinden oder in einer kontrollierten Atmosphäre. Kontrollierte Atmosphären sind beispielsweise Schutzgas-Atmosphären, reduzierende und/oder oxidierende Atmosphären.

Demgemäß beschreibt die vorliegende Erfindung auch ein Verfahren zur Herstellung eines Formkörpers, wie oben beschrieben, umfassend die Schritte
(I) Herstellen eines Gemischs, enthaltend ein wie oben beschriebenes zeolithisches Material oder ein zeolithisches Material, erhältlich gemäß einem wie oben beschriebenen Verfahren, und mindestens ein Bindermaterial;
(II) Kneten des Gemischs;
(III) Verformen des gekneteten Gemischs unter Erhalt mindestens eines Formkörpers.

Die Form der erfindungsgemäß hergestellten Formkörper kann beliebig gewählt werden. Insbesondere sind unter anderem Kugeln, ovale Formen, Zylinder oder Tabletten möglich.

Besonders bevorzugt wird im Rahmen der vorliegenden Erfindung das Verformen durch Extrusion des gemäß (II) erhaltenen gekneteten Gemischs durchgeführt, wobei als Extrudate weiter bevorzugt im wesentlichen zylinderförmige Stränge mit einem Durchmesser im Bereich von 1 bis 20 mm, weiter bevorzugt im Bereich von 1 bis 10 mm, weiter bevorzugt im Bereich von 2 bis 10 mm und insbesondere bevorzugt im Bereich von 2 bis 5 mm erhalten werden.

Dem Schritt (III) schließt sich im Rahmen der vorliegenden Erfindung bevorzugt mindestens ein Trocknungsschritt an. Dieser mindestens eine Trocknungsschritt erfolgt dabei bei Temperaturen im Bereich von im allgemeinen 80 bis 160°C, bevorzugt von 90 bis 145 °C und besonders bevorzugt von 100 bis 130°C, wobei die Trocknungsdauer im Allgemeinen bei 6 h oder mehr, beispielsweise im Bereich von 6 bis 24, liegt. Es sind jedoch in Abhängigkeit vom Feuchtigkeitsgehalt des zu trocknenden Materials auch kürzere Trockenzeit wie beispielsweise ungefähr 1, 2, 3, 4, oder 5 h möglich.

Vor und/oder nach dem Trocknungsschritt kann das bevorzugt erhaltene Extrudat beispielsweise zerkleinert werden. Dabei wird vorzugsweise ein Granulat oder Splitt mit einem Partikeldurchmesser von 0,1 bis 5 mm, insbesondere 0,5 bis 2 mm erhalten.

Demgemäß beschreibt die vorliegende Erfindung auch ein Verfahren zur Herstellung eines Formkörpers, wie oben beschrieben, umfassend die Schritte
(I) Herstellen eines Gemischs, enthaltend ein wie oben beschriebenes zeolithisches Material oder ein zeolithisches Material, erhältlich gemäß einem wie oben beschriebenen Verfahren, und mindestens ein Bindermaterial;
(II) Kneten des Gemischs;
(III) Verformen des gekneteten Gemischs unter Erhalt mindestens eines Formkörpers;
(IV) Trocknen des mindestens einen Formkörpers.

Dem Schritt (IV) schließt sich im Rahmen der vorliegenden Erfindung bevorzugt mindestens ein Calcinierungsschritt an. Die Calcinierung wird bei Temperaturen im Bereich von im allgemeinen 350 bis 750°C und bevorzugt von 450 bis 600 °C durchgeführt.

Die Calcinierung kann unter jeder geeigneten Gasatmosphäre erfolgen, wobei Luft und/oder Magerluft bevorzugt sind. Weiter wird die Calcinierung bevorzugt in einem Muffelofen, einem Drehrohrofen und/oder einem Bandkalzinierofen durchgeführt, wobei die Calcinierungsdauer im Allgemeinen bei 1 h oder mehr, beispielsweise im Bereich von 1 bis 24 oder im Bereich von 3 bis 12 liegt. Demgemäß ist es im Rahmen des erfindungsgemäßen Verfahrens beispielsweise möglich, den Formkörper einmal, zweimal oder öfter für jeweils mindestens 1 h wie beispielsweise jeweils im Bereich von 3 bis 12 h zu calcinieren, wobei die Temperaturen während eines Calcinierungsschrittes gleich bleiben oder kontinuierlich oder diskontinuierlich geändert werden können. Wird zweimal oder öfter calciniert, können sich die Calcinierungstemperaturen in den einzelnen Schritten unterscheiden oder gleich sein.

Demgemäß betrifft die vorliegende Erfindung auch ein Verfahren zur Herstellung eines Formkörpers, wie oben beschrieben, umfassend die Schritte
(I) Herstellen eines Gemischs, enthaltend ein wie oben beschriebenes zeolithisches Material oder ein zeolithisches Material, erhältlich gemäß einem wie oben beschriebenen Verfahren, und mindestens ein Bindermaterial;
(II) Kneten des Gemischs;
(III) Verformen des gekneteten Gemischs unter Erhalt mindestens eines Formkörpers;
(IV) Trocknen des mindestens einen Formkörpers;
(V) Calcinieren des mindestens einen getrockneten Formkörpers.

Nach dem Calcinierungsschritt kann das calcinierte Material beispielsweise zerkleinert werden. Dabei wird vorzugsweise ein Granulat oder Splitt mit einem Partikeldurchmesser von 0,1 bis 5 mm, insbesondere 0,5 bis 2 mm erhalten.

Vor und/oder nach dem Trocknen und/oder vor und/oder nach dem Calcinieren kann der mindestens eine Formkörper mit einer konzentrierten oder verdünnten Broenstedt-Säure oder einem Gemisch aus zwei oder mehr Broenstedt-Säuren behandelt werden. Geeignete Säuren sind beispielsweise Salzsäure, Schwefelsäure, Phosphorsäure, Salpetersäure oder Carbonsäuren, Dicarbonsäuren oder Oligo- oder Polycarbonsäuren wie beispielsweise Nitrilotriessigsäure, Sulfosalicylsäure oder Ethylendiaminotetraessigsäure.

Bevorzugt schließt sich an diese mindestens eine Behandlung mit mindestens einer Broenstedtsäure mindestens ein Trockenschritt und/oder mindestens ein Calcinierungsschritt an, der jeweils unter den oben beschriebenen Bedingungen durchgeführt wird.

Gemäß einer weiteren bevorzugten Ausführungsform des erfindungsgemäßen Verfahrens können die Katalysatorstränge zur besseren Härtung einer Wasserdampfbehandlung unterzogen werden, nach der bevorzugt nochmals mindestens einmal getrocknet und/oder mindestens einmal calciniert wird. Beispielsweise wird nach mindestens einem Trockenschritt und mindestens einem nachfolgenden Calcinierschritt der calcinierte Formkörper der Wasserdampfbehandlung unterzogen und anschließend nochmals mindestens einmal getrocknet und/oder mindestens einmal calciniert.

Die erfindungsgemäß erhaltenen Formkörper weisen Härten auf, die im Allgemeinen im Bereich von 2 bis 15 N, bevorzugt im Bereich von 5 bis 15 N und besonders bevorzugt im Bereich von 10 bis 15 N liegen.

Demgemäß betrifft die vorliegende Erfindung auch einen Formkörper, wie oben beschrieben, mit einer Schneidhärte im Bereich von 2 bis 15 N.

Die obenstehend beschriebene Härte wurde im Rahmen der vorliegenden Erfindung an einem Apparat der Firma Zwick, Typ BZ2.5/TS1S mit einer Vorkraft von 0,5 N, einer Vorkraftschubgeschwindigkeit von 10 mm/min und einer nachfolgenden Prüfgeschwindigkeit von 1,6 mm/min bestimmt. Das Gerät besaß einen festsitzenden Drehteller und einen frei beweglichen Stempel mit eingebauter Schneide von 0,3 mm Stärke. Der bewegliche Stempel mit der Schneide war mit einer Kraftmessdose zur Kraftaufnahme verbunden und bewegte sich während der Messung gegen den festsitzenden Drehteller hin, auf dem der zu untersuchende Katalysatorformkörper lag. Das Prüfgerät wurde über einen Computer gesteuert, der die Messergebnisse registrierte und auswertete. Die erzielten Werte stellen den Mittelwert aus den Messungen zu jeweils 10 Katalysatorformkörpern dar. Die Katalysatorformkörper wiesen eine zylindrische Geometrie auf, wobei ihre mittlere Länge in etwa dem Zwei- bis Dreifachen des Durchmessers entsprach, und wurden dabei mit der Schneide von 0,3 mm Stärke mit zunehmender Kraft solange belastet, bis der Formkörper durchtrennt war. Die Schneide wurde dabei senkrecht zur Längsachse des Formkörpers auf den Formkörper aufgebracht. Die dazu benötigte Kraft ist die Schneidhärte (Einheit N).

Demgemäß betrifft die vorliegende Erfindung auch einen Formkörper, erhältlich durch ein Verfahren gemäß einer der oben beschriebenen Ausführungsformen.

Der erfindungsgemäße mindestens eine Formkörper oder/und der erfindungsgemäß hergestellte Formkörper kann generell in sämtlichen Verfahren oder Arbeitsschritten eingesetzt werden, in denen die Eigenschaften des Formkörpers und insbesondere des im Formkörper enthaltenen erfindungsgemäßen oder erfindungsgemäß hergestellten zeolithischen Materials erwünscht sind. Ganz besonders bevorzugt wird der erfindungsgemäße mindestens eine Formkörper oder der erfindungsgemäß hergestellte Formkörper als Katalysator bei chemischen Umsetzungen eingesetzt.

Das erfindungsgemäße zeolithische Material und/oder die erfindungsgemäßen Formkörper wird/werden beispielsweise bevorzugt in der Herstellung von epsilon-Caprolactam aus Cyclohexanonoxim und der Herstellung von N-Vinylpyrrolidon aus N-Hydroxyethylpyrrolidon eingesetzt.

Ganz besonders bevorzugt ist die Verwendung des zeolithischen Materials und/oder der Formkörper bei der selektiven Synthese von Triethylendiamin (TEDA).

Demgemäß betrifft die vorliegende Erfindung auch die wie oben beschriebene Verwendung als Katalysator, wobei der Katalysator zur selektiven Synthese von Triethylendiamin eingesetzt wird.

TEDA (IUPAC-Name: 1,4-Diazabicyclo(2,2,2)-octan) ist ein wichtiges Zwischen- und Endprodukt in der chemischen Industrie, das hauptsächlich als solches bei der Polyurethanherstellung als Katalysator eingesetzt wird. Zur Herstellung von TEDA existiert eine große Anzahl verschiedener Synthesen, die sich hauptsächlich in der Wahl der Edukte und der benutzten Katalysatoren unterscheiden.

Zur TEDA-Herstellung ist der Einsatz einer Palette verschiedener Edukte möglich, die einen C2-Baustein und/oder einen Stickstoffbaustein enthalten und cyclisch oder acyclisch sein können. Beispiele geeigneter Edukte umfassen Ethylendiamin, Diethylentriamin, Ethanolamin, Aminoethylethanolamin, Piperazin, Aminoethylpiperazin und Hydroxyethylpiperazin. Es wird häufig ein einziges Edukt eingesetzt, wobei sich jedoch auch Gemische von zwei oder mehr geeigneten Edukten vorteilhaft verwenden lassen. Üblicherweise wird dem Reaktionsgemisch noch Wasser zugesetzt. Durch die Wahl der Edukte wird die Zusammensetzung des Produktgemischs entscheidend beeinflusst, wobei insbesondere die Vermeidung der Bildung von Nebenprodukten, neben der Verfügbarkeit der Ausgangsprodukte, ein wichtiger Aspekt im Hinblick auf die zu erreichende Spezifikation in der Aufarbeitung ist. In den meisten Fällen wird zur Erhöhung der Selektivität bezüglich des gewünschten Produktes TEDA die Synthese so durchgeführt, dass lediglich ein Teilumsatz des oder der eingesetzten Edukte eintritt. Der Nachteil der geringen Ausbeute wird wegen der erreichbaren niedrigen Mengen an unerwünschten Nebenprodukten in Kauf genommen.

Ganz allgemein betrifft die vorliegende Erfindung daher ein Verfahren zur selektiven Herstellung von Triethylendiamin durch Umsetzung mindestens eines Eduktes, das eine Struktureinheit (I) 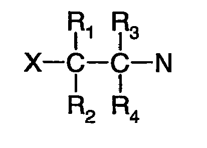 aufweist, wobei R₁, R₂, R₃ und R₄ unabhängig voneinander ein Wasserstoffatom oder eine Alkylgruppe mit 1 bis 4 Kohlenstoffatomen und X ein Sauerstoff- oder Stickstoffatom darstellen.

Beispiele für solche Verbindungen sind unter anderem Ethylendiamin (EDA), Monoethanolamin, Diethanolamin, Triethanolamin, Piperazin (PIP), Diethylentriamin, Triethylentetramin, Tri(2-aminoethyl)amin, N-(2-aminoethyl)ethanolamin, Morpholin, N-(2-hydroxyethyl)piperazin, N,N'-Bis(2-hydroxyethyl)piperazin, N-(2-aminoethyl)piperazin und N,N'-Bis(2-aminoethyl)piperazin.

Im Rahmen der vorliegenden Erfindung ist es beispielsweise bevorzugt möglich, TEDA dadurch herzustellen, dass als Edukt Piperazin (PIP) eingesetzt wird. Ebenso ist es möglich, als Edukt Ethylendiamin (EDA) einzusetzen. Möglich ist auch, ein Gemisch aus EDA und PIP als Edukt einzusetzen.

Daher betrifft die vorliegende Erfindung ein Verfahren zur selektiven Herstellung von Triethylendiamin durch Umsetzung eines Eduktes, bestehend aus
(A) x Gew.-% Piperazin und
(B) y Gew.% Ethylendiamin,
wobei x + y = 100 und 0 ≤ x ≤ 100 und 0 ≤ y ≤ 100, an einem Zeolithkatalysator, wobei der Zeolithkatalysator ein zeolithisches Material gemäß einem der Ansprüche 1 bis 4 oder ein zeolithisches Material, erhältlich gemäß einem der Ansprüche 10 bis 14, enthält.

Das erfindungsgemäße Verfahren kann diskontinuierlich durchgeführt werden und wird bevorzugt kontinuierlich durchgeführt.

Die erfindungsgemäße Umsetzung kann in der Flüssigphase durchgeführt werden und wird bevorzugt in der Gasphase durchgeführt.

Bevorzugt wird die Umsetzung in Gegenwart mindestens eines Lösungs- oder Verdünnungsmittels durchgeführt.

Als Lösungs- oder Verdünnungsmittel eignen sich z.B. acyclische oder cyclische Ether mit 2 bis 12 Kohlenstoffatomen, wie Dimethylether, Diethylether, Di-n-Propylether oder dessen Isomere, MTBE, THF, Pyran, oder Lactone, wie gamma-Butyrolacton, Polyether, wie Monoglyme, Diglyme etc., aromatische oder aliphatische Kohlenwasserstoffe, wie Benzol, Toluol, Xylol, Pentan, Cyclopentan, Hexan und Petrolether, oder deren Gemische und besonders auch N-Methylpyrrolidon (NMP) oder Wasser oder wässrige organische Lösungs- oder Verdünnungsmittel der oben genannten Art. Weiterhin ist Ammoniak als Lösungs- oder Verdünnungsmittel geeignet.

Besonders bevorzugt wird als Lösungs- oder Verdünnungsmittel, insbesondere Lösungsmittel, Wasser eingesetzt.

Als Verdünnungsmittel bei der Durchführung der Umsetzung in der Gasphase sind auch Inertgase wie Stickstoff (z.B. über die Sättigung des Reaktorzulaufs hinaus) oder Argon geeignet. Bevorzugt wird die Umsetzung in der Gasphase in Gegenwart von Ammoniak durchgeführt.

Die Eduktkomponenten oder der Reaktorzulauf werden vorteilhafterweise vortemperiert.

Als Reaktoren, in denen das erfindungsgemäße Verfahren durchgeführt wird, eignen sich Rührbehälter, insbesondere Rohrreaktoren und Rohrbündelreaktoren.

Der Zeolith-Katalysator, bevorzugt als Formkörper, ist im Reaktor bevorzugt als Festbett angeordnet.

Die Umsetzung in der Flüssigphase kann z.B. in der Suspensions-, Riesel- oder Sumpffahrweise erfolgen.

Die bevorzugte Umsetzung in der Gasphase kann in einem Katalysator-Wirbelbett oder bevorzugt -Festbett erfolgen.

Im Falle, dass als Edukt Piperazin allein eingesetzt wird, sind Verfahrensführungen bevorzugt, bei denen die Reaktionstemperatur im Bereich von 300 bis 450 °C und besonders bevorzugt im Bereich von 330 bis 400 °C liegt. Dabei liegt der Druck, unter dem die Umsetzung stattfindet, in einem Bereich von 0,01 bis 50 bar, bevorzugt im Bereich von 0,5 bis 20 bar und insbesondere bevorzugt im Bereich des Atmosphärendruckes, zuzüglich des Druckverlustes, der beim Durchgang über das Katalysatorbett entsteht.

Piperazin wird weiter besonders bevorzugt im Gemisch mit Wasser eingesetzt, wobei weiter bevorzugt ein Eduktstrom verwendet wird, der mindestens 10 Gew. -% Wasser, bevorzugt 10 bis 60 Gew.-% Wasser und insbesondere 20 bis 60 Gew.-% Wasser enthält, jeweils bezogen auf das Gesamtgewicht des Eduktstroms, enthaltend Piperazin- und Wasser.

Im Falle, dass Piperazin als einziges Edukt eingesetzt wird, ist eine WHSV (weight hourly space velocity), bezogen auf in die Umsetzung eingesetztes Piperazin, im Bereich von 0,01 bis 5 h⁻¹, vorzugsweise im Bereich von 0,02 bis 1 h⁻¹ und besonders bevorzugt im Bereich von 0,05 bis 0,8 h⁻¹ bevorzugt.

Im Falle, dass als Edukt EDA allein eingesetzt wird, sind Verfahrensführungen bevorzugt, bei denen die Reaktionstemperatur im Bereich von 300 bis 400 °C und besonders bevorzugt im Bereich von 320 bis 350 °C liegt. Dabei liegt der Druck, unter dem die Umsetzung stattfindet, in einem Bereich von 0,01 bis 50 bar, bevorzugt im Bereich von 0,5 bis 20 bar und insbesondere bevorzugt im Bereich des Atmosphärendruckes, zuzüglich des Druckverlustes, der beim Durchgang über das Katalysatorbett entsteht.

EDA wird weiter besonders bevorzugt im Gemisch mit Wasser eingesetzt, wobei weiter bevorzugt ein Eduktstrom verwendet wird, der höchstens 50 Gew. % Wasser, bevorzugt höchstens 30 Gew.-% Wasser und insbesondere höchstens 10 Gew.-% Wasser enthält, jeweils bezogen auf das Gesamtgewicht des Eduktstroms, enthaltend EDA und Wasser.

Im Falle, dass EDA als einziges Edukt eingesetzt wird, ist eine WHSV (weight hourly space velocity), bezogen auf in die Umsetzung eingesetztes EDA, im Bereich von 0,01 bis 5 h⁻¹, vorzugsweise im Bereich von 0,02 bis 1 h⁻¹ und besonders bevorzugt im Bereich von 0,05 bis 0,8 h⁻¹ bevorzugt.

Im Falle, dass ein Gemisch aus PIP und EDA als Edukt eingesetzt wird, wird die Reaktion bevorzugt so geführt, dass bei kontinuierlichem Betrieb im stationären Zustand 10 bis 50 Gew.-% Wasser und 90 bis 50 Gew.-% PIP und EDA (Summe Gew.-%-Anteile der beiden Verbindungen PIP und EDA), weiter bevorzugt 30 bis 50 Gew.-% Wasser und 70 bis 50 Gew.-% PIP und EDA und insbesondere bevorzugt 40 bis 50 Gew.-% Wasser und 60 bis 50 Gew.-% PIP und EDA zugeführt werden, wobei der Anteil des PIP oder des EDA gegebenenfalls zu Gunsten oder zu Lasten des EDA oder des PIP erniedrigt oder erhöht werden kann.

Bevorzugt wird in diesem Fall die Reaktion so geführt, dass der Eduktstrom im kontinuierlichen Betrieb EDA und PIP im Gewichtsverhältnis im Bereich von 1:1 bis 10:1, weiter bevorzugt im Bereich von 2:1 bis 6:1 und insbesondere bevorzugt im Bereich von 3:1 bis 5:1, jeweils gerechnet als Gewicht EDA zu Gewicht PIP, enthält.

Im Rahmen der wie oben beschriebenen Ausführungsform, gemäß der 35 bis 60 Gew.-%, beispielsweise etwa 40 Gew.-%, EDA zugegeben werden, kann die Umsetzung im stationären Zustand so geführt werden, dass sich EDA im wesentlichen vollständig zu TEDA und PIP umsetzt, wobei PIP dem Produktstrom zusammen mit gegebenenfalls zusätzlich vorhandenen Zwischen- und/oder Nebenprodukten bevorzugt destillativ entzogen wird und, gegebenenfalls nach Abtrennung mindestens eines dieser Zwischen-und/oder Nebenprodukte, mit in etwa derselben Menge an EDA versetzt wird und das resultierende Gemisch, enthaltend EDA und PIP, der Umsetzung wieder zugeführt wird.

Diese Verfahrensvariante wird bevorzugt so durchgeführt, dass der Verbrauch an PIP in der Bilanz gegen Null geht, während des kontinuierlichen Betriebs folglich im Wesentlichen kein zusätzliches PIP zugegeben wird.

Bei dieser Verfahrensführung hat sich überraschend gezeigt, dass die Menge an ausgetragenem EDA gegen Null geht. Die Auftrennung des Reaktoraustrags ist daher besonders einfach.

Ein besonderer Vorteil des Verfahrens besteht darin, dass man Zwischenfraktionen, die sowohl TEDA als auch PIP enthalten, erneut der Umsetzung zuführen kann.

Im Falle, dass ein Gemisch aus EDA und PIP als Edukt eingesetzt wird, sind Verfahrensführungen bevorzugt, bei denen die Reaktionstemperatur im Bereich von 290 bis 400 °C, bevorzugt im Bereich von 310 bis 370 °C und besonders bevorzugt im Bereich von 310 bis 350 °C liegt. Dabei liegt der Druck, unter dem die Umsetzung stattfindet, in einem Bereich von 0,1 bis 10 bar, bevorzugt im Bereich von 0,8 bis 2 bar und insbesondere bevorzugt im Bereich des Atmosphärendruckes, zuzüglich des Druckverlustes, der beim Durchgang über das Katalysatorbett entsteht.

Im Falle, dass ein Gemisch aus EDA und PIP als Edukt eingesetzt wird, ist eine WHSV (weight hourly space velocity), bezogen auf die in die Umsetzung eingesetzten Amine, im Bereich von 0,05 bis 6 h⁻¹, vorzugsweise im Bereich von O,2 bis 2 h⁻¹ und besonders bevorzugt im Bereich von 0,3 bis 1 h⁻¹ bevorzugt.

Die Verwendung des erfindungsgemäßen Katalysators, bevorzugt in Form von Formkörpern, zeichnet sich unter anderem dadurch aus, dass eine sehr hohe Standzeit des Katalysators erreicht wird. Diese liegt im allgemeinen im Bereich von mehr als 1000 h, bevorzugt bei mindestens 1200 h, weiter bevorzugt bei mindestens 1400 Stunden, weiter bevorzugt bei mindestens 1600 h, weiter bevorzugt bei mindestens 1800 h und insbesondere bevorzugt bei mindestens 2000 h. Bei konstanten Reaktionsparametern wurde während der oben angegebenen Standzeiten keine Verschlechterung des Reaktionsumsatzes beobachtet.

Demgemäß beschreibt die vorliegende Erfindung auch ein Verfahren, wie oben beschrieben, wobei der Katalysator eine Standzeit von mindestens 1200 h aufweist. Ganz besonders bevorzugt wird der Katalysator zumindest teilweise in der H-Form eingesetzt. Wird ein Teil des Katalysators nicht in der H-Form eingesetzt, so wird dieser Teil ganz besonders bevorzugt in der NH₄⁺-Form eingesetzt. Insbesondere bevorzugt wird der gesamte Katalysator in der H-Form eingesetzt.

Demgemäß betrifft die vorliegende Erfindung auch ein Verfahren, wie oben beschrieben, wobei mindestens ein Teil des Katalysators in der H-Form eingesetzt wird.

In einer weiteren Ausführungsform des erfindungsgemäßen Verfahrens wird der Katalysator nach dem Einsatz unabhängig von seiner Form, z.B. nach Abnahme der Aktivität und/oder der Selektivität, durch ein Verfahren regeneriert, bei dem die Regenerierung durch gezieltes Abbrennen der für die Deaktivierung verantwortlichen Beläge erfolgt. Dabei wird bevorzugt in einer Inertgasatmosphäre gearbeitet, die genau definierte Mengen an Sauerstoff liefernden Substanzen enthält. Ein solches Regenerierungsverfahren ist unter anderem in der WO 98/55228 und der DE 197 23 949 A1 beschrieben, deren diesbezügliche Offenbarung durch Bezugnahme hiermit vollumfänglich in den Gegenstand der vorliegenden Anmeldung einbezogen wird.

Nach der Regeneration sind die Aktivität und/oder die Selektivität des Katalysators, verglichen mit dem Zustand unmittelbar vor der Regeneration, erhöht.

Der zu regenerierende, erfindungsgemäß eingesetzte Zeolith-Katalysator wird entweder in der Umsetzungsvorrichtung (Reaktor) oder in einem externen Ofen in einer Atmosphäre, die 0,1 bis ungefähr 20 Volumen-Anteile von Sauerstoff liefernden Substanzen, besonders bevorzugt 0,1 bis 20 Volumenanteile Sauerstoff, enthält, auf eine Temperatur im Bereich von 250°C bis 800 °C, vorzugsweise von 400 °C bis 550°C und insbesondere von 450 °C bis 500 °C aufgeheizt. Dabei wird das Aufheizen vorzugsweise mit einer Aufheizrate von 0,1 °C/min bis 20 °C/min, vorzugsweise von 0,3 °C/min bis 15 °C/min und insbesondere von 0,5 °C/min bis 10 °C/min durchgeführt.

Während dieser Aufheizphase wird der Katalysator bis zu einer Temperatur aufgeheizt, bei der die sich dort befindlichen, meist organischen Beläge zu zersetzen beginnen, während gleichzeitig die Temperatur über den Sauerstoffgehalt geregelt wird und somit nicht derart ansteigt, dass es zu Schädigungen der Katalysatorstruktur kommt. Das langsame Erhöhen der Temperatur bzw. das Verweilen bei niedriger Temperatur durch Einstellen des entsprechenden Sauerstoffgehaltes und der entsprechenden Heizleistung ist bei hohen organischen Beladungen des zu regenerierenden Katalysators ein wesentlicher Schritt zur Verhinderung einer lokalen Überhitzung des Katalysators. Sinkt die Temperatur des Abgasstroms am Reaktorausgang trotz steigender Mengen an Sauerstoff liefernden Substanzen im Gasstrom, so ist das Abbrennen der organischen Beläge beendet. Die Dauer der Behandlung beträgt im Allgemeinen jeweils 1 bis 30, vorzugsweise ungefähr 2 bis ungefähr 20 und insbesondere ungefähr 3 bis ungefähr 10 Stunden.

Das anschließende Abkühlen des so regenerierten Katalysators wird bevorzugt so durchgeführt, dass das Abkühlen nicht zu schnell erfolgt, da sonst die mechanische Festigkeit des Katalysators negativ beeinflusst werden kann.

Es kann erforderlich sein, den Katalysator nach der durchgeführten Regeneration durch Calcinieren, wie oben beschrieben, einer Spülung mit Wasser und/oder verdünnten Säuren wie beispielsweise Salzsäure zu unterziehen, um die durch Verunreinigung der Edukte gegebenenfalls verbleibende anorganische Beladung des Katalysators (Alkalispuren etc.) zu entfernen. Anschließend kann eine erneute Trocknung und/oder ein erneutes Calcinieren des Katalysators durchgeführt werden.

In einer weiteren Ausführungsform des erfindungsgemäßen Verfahrens wird der zumindest teilweise deaktivierte Katalysator vor dem Aufheizen gemäß der Regenerationsprozedur mit einem Lösungsmittel im Umsetzungsreaktor oder in einem externen Reaktor gewaschen, um noch anhaftendes Wertprodukt zu entfernen. Dabei wird das Waschen so durchgeführt, dass zwar die jeweils am Katalysator anhaftenden Wertprodukte von diesem entfernt werden können, aber Temperatur und Druck nicht so hoch gewählt werden, dass die meist organischen Beläge ebenfalls entfernt werden. Vorzugsweise wird der Katalysator dabei mit einem geeigneten Lösungsmittel lediglich gespült. Somit eignen sich für diesen Waschvorgang alle Lösungsmittel, in denen sich das jeweilige Umsetzungsprodukt gut löst. Die benutzte Menge an Lösungsmittel sowie die Dauer des Waschvorgangs sind nicht kritisch. Der Waschvorgang kann mehrmals wiederholt und bei erhöhter Temperatur durchgeführt werden. Bei Verwendung von CO₂ als Lösungsmittel ist überkritischer Druck bevorzugt, ansonsten kann der Waschvorgang unter Normaldruck bzw. erhöhtem oder überkritischem Druck erfolgen. Nach der Beendigung des Waschvorgangs wird der Katalysator im Allgemeinen getrocknet. Obwohl der Trocknungsvorgang im allgemeinen unkritisch ist, sollte die Trocknungstemperatur die Siedetemperatur des zum Waschen verwendeten Lösungsmittels nicht zu stark übersteigen, um ein schlagartiges Verdampfen des Lösungsmittels in den Poren, insbesondere in den Mikroporen zu vermeiden, da auch dies zu Schädigungen des Katalysators führen kann.

Eine bevorzugte Ausführung des Herstellverfahrens besteht darin, dass das erfindungsgemäße, kontinuierliche Verfahren zur Synthese von TEDA bei der Regeneration des erfindungsgemäßen Katalysators nicht unterbrochen werden muss, um so den Verfahrensdurchsatz zu steigern. Dies kann durch die Verwendung von mindestens zwei parallel verschalteten Reaktoren erreicht werden, die wechselweise betrieben werden können.

Die Katalysatorregeneration kann derart durchgeführt werden, dass mindestens einer der parallel geschalteten Reaktoren aus der jeweiligen Reaktionsstufe abgekoppelt wird und der in diesem Reaktor enthaltene Katalysator regeneriert wird, wobei im Laufe des kontinuierlichen Verfahrens in jeder Stufe immer mindestens ein Reaktor zur Umsetzung des Eduktes oder der Edukte zur Verfügung steht.

Das erfindungsgemäß erhaltene TEDA kann zur Verbesserung seiner Reinheit aus geeigneten Lösungsmitteln wie beispielsweise Pentan oder Hexan umkristallisiert werden. Meist ist dies jedoch nicht erforderlich, da TEDA nach dem erfindungsgemäßen Verfahren mit Reinheiten von mindestens 95 Gew.-%, bevorzugt mindestens 96 Gew.-% und besonders bevorzugt von mindestens 97 Gew.-%, hergestellt werden kann.

In einer besonderen Ausgestaltung wird das anspruchsgemäße TEDA-Herstellverfahren kombiniert mit dem sich anschließenden TEDA-Verfahren gemäß DE 199 33 850 A1.

Gemäß dieser Kombination wird zunächst TEDA wie oben beschrieben hergestellt. Bei der sich anschließenden Aufarbeitung des TEDAs (z.B. destillativ), die mehrstufig sein kann, wird das TEDA, bevorzugt in der letzten Aufarbeitungsstufe (insbesondere Destillations- bzw. Rektifikationsstufe), verdampft und das, z.B. am Kopf oder in einem Seitenabzug der Destillationskolonne erhaltene, dampfförmige TEDA, das bevorzugt eine Reinheit von größer 95 Gew.-%, insbesondere von größer 97 Gew.-% besitzt, in ein flüssiges Lösungsmittel eingeleitet. Diese Einleitung des dampfförmigen TEDAs direkt in ein flüssiges Lösungsmittel wird im Folgenden auch 'TEDA-Quench' genannt.

Die Einleitung des dampfförmigen TEDAs in das flüssige Lösungsmittel erfolgt in einem Quenchapparat, z.B. bevorzugt in einem Fallfilmkondensator (Dünnschicht-, Rieselfilm- oder Fallstromkondensator) oder in einem Düsenapparat. Dabei kann das dampfförmige TEDA im Gleich- oder im Gegenstrom mit dem flüssigen Lösungsmittel geführt werden. Vorteilhaft ist die Einleitung des dampfförmigen TEDAs von oben in den Quenchapparat. Weiterhin vorteilhaft ist die tangentiale Zufuhr des flüssigen Lösungsmittels am Kopf des Fallfilmkondensators oder die Zufuhr des flüssigen Lösungsmittels durch eine oder mehrere Düsen, um eine vollständige Benetzung der Innenwand des Quenchapparates zu erreichen.

Im Allgemeinen wird die Temperatur im TEDA-Quench durch Temperierung des eingesetzten Lösungsmittels und/oder des Quenchapparates auf 20 bis 100°C, bevorzugt 30 bis 60°C, eingestellt. Der Absolutdruck im TEDA-Quench beträgt im Allgemeinen 0,5 bis 1,5 bar.

Im Allgemeinen wird so verfahren, dass, je nach Art des Lösungsmittels, beim TEDA-Quench zunächst Lösungen mit einem TEDA-Gehalt von ca. 1 bis 50 Gew.-%, bevorzugt 20 bis 40 Gew.-%, erhalten werden.

Durch anschließende Auskristallisation des TEDAs aus der so erhaltenen Lösung wird reines TEDA mit hoher Qualität erhalten.

Das flüssige Lösungsmittel wird im allgemeinen aus der Gruppe cyclische oder acyclische Kohlenwasserstoffe, chlorierte aliphatische Kohlenwasserstoffe, aromatische Kohlenwasserstoffe, Alkohole, Ketone, aliphatische Carbonsäureester, aliphatische Nitrile und Ether ausgewählt.

Zur Herstellung einer Lösung von reinem TEDA gemäß obiger Verfahrenskombination, die z.B. als Katalysatorlösung bei der Polyurethanschaumherstellung verwendet werden kann, wird als Lösungsmittel für den TEDA-Quench bevorzugt ein Alkohol (z.B. Ethylenglykol, 1,4-Butandiol, bevorzugt Dipropylenglykol) eingesetzt. Die Farbzahl einer so erhaltenen 33 Gew.-%igen TEDA-Lösung in Dipropylenglykol beträgt kleiner 150 APHA, insbesondere kleiner 100 APHA, ganz besonders kleiner 50 APHA.

Die so erhaltenen Lösungen sind bezüglich der Farbzahl im Allgemeinen mehr als 6 Monate, bevorzugt mehr als 12 Monate und insbesondere bevorzugt mehr als 24 Monate lagerstabil.

Zur Herstellung von reinem (kristallinem) TEDA gemäß obiger Verfahrenskombination wird als Lösungsmittel für den TEDA-Quench bevorzugt ein aliphatischer Kohlenwasserstoff, insbesondere ein gesättigter aliphatischer Kohlenwasserstoff mit 5 bis 8 C-Atomen (wie z.B. Hexan, Heptan, bevorzugt Pentan) verwendet. Die Kristallisation des reinen TEDAs aus der erfindungsgemäß hergestellten TEDA-Lösung kann nach den dem Fachmann bekannten Verfahren erfolgen. Die durch eine nachfolgende mehrstufige, oder bevorzugt einstufige, Kristallisation erhaltenen TEDA-Kristalle sind hochrein (Reinheit von im Allgemeinen mindestens 99,5 Gew.-%, insbesondere mindestens 99,8 Gew.-%. Gehalt an PIP kleiner 0,1 Gew.-%, insbesondere kleiner 0,05 Gew.-%, Gehalt an N-Ethylpiperazin kleiner 0,02 Gew.-%, insbesondere kleiner 0,01 Gew.-%) und die Farbzahl einer 33 gew.-%igen Lösung in Dipropylenglykol beträgt kleiner 50 APHA, insbesondere kleiner 30 APHA.

Alle APHA-Zahlen sind gemäß DIN ISO 6271 bestimmt.

Die Erfindung wird anhand der folgenden Beispiele und der Figuren näher illustriert.

### Figurenbeschreibung

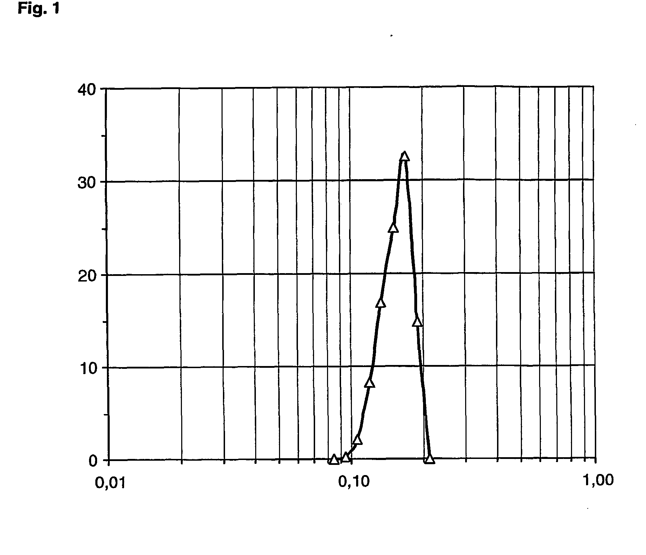

- Fig. 1: zeigt die Partikelgrößenverteilung des gemäß Beispiel 1 hergestellten Zeolith- Materials. Dabei wurde die Partikelgrößenverteilung mit Hilfe von Laserbeu- gung am Gerät "Mastersizer 2000" (Modul Hydro 2000G) der Firma Malvern Instruments Ltd. gemäß DIN 13320 gemessen. Dabei wurde als zu untersu- chende Lösung eine 0,14 Gew.-%ige Lösung von ZSM-5-Kristallen in deioni- siertem Wasser bei Raumtemperatur vermessen. Diese Lösung wurde herge- stellt durch Verdünnen einer gemäß Beispiel 1 erhaltenen Lösung, die vor der Behandlung mit HNO₃ aus dem Kristallisationsschritt resultierte. Der Bre- chungsindex der derart verdünnten Lösung betrug 1,503. Die Partikel zeigen nach Fig. 1 zeigt eine mittlere Größe von 0,15 µm bei einem Variationskoeffi- zienten von 21 %. Auf der Rechtswertachse des Diagramms in Fig. 1 ist die Partikelgröße in der Einheit [µm] aufgetragen, auf der Hochwertachse der je- weilige prozentuale Anteil der Teilchen in [%].

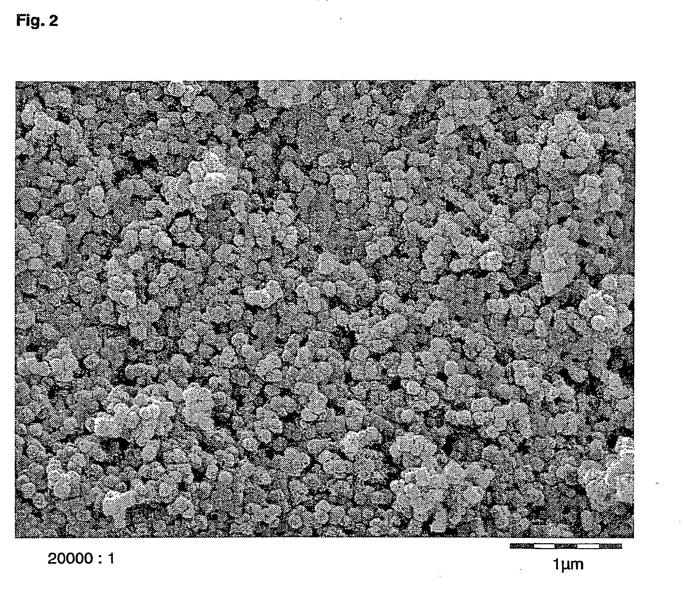

- Fig. 2: zeigt mit einer Auflösung von 20.000 : 1 eine SEM-Aufnahme des gemäß Beispiel 1 erhaltenen ZSM 5-Pulvers.

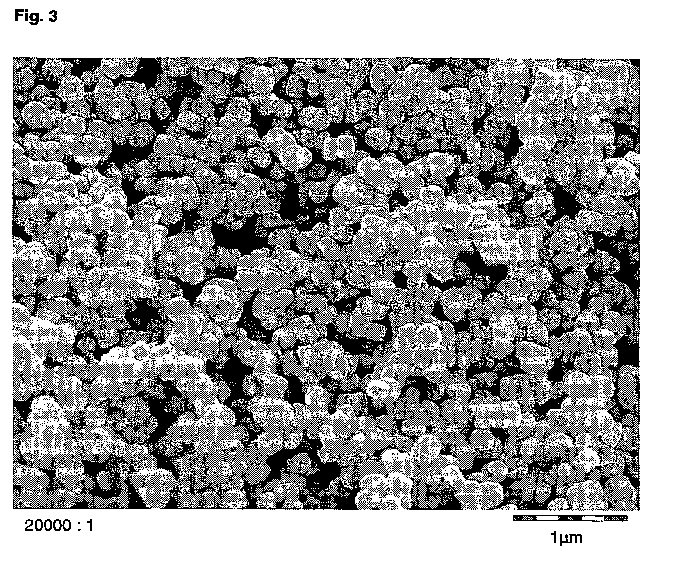

- Fig. 3: zeigt mit einer Auflösung von 20.000 : 1 eine SEM-Aufnahme des gemäß Beispiel 4 erhaltenen ZSM 5-Pulvers.

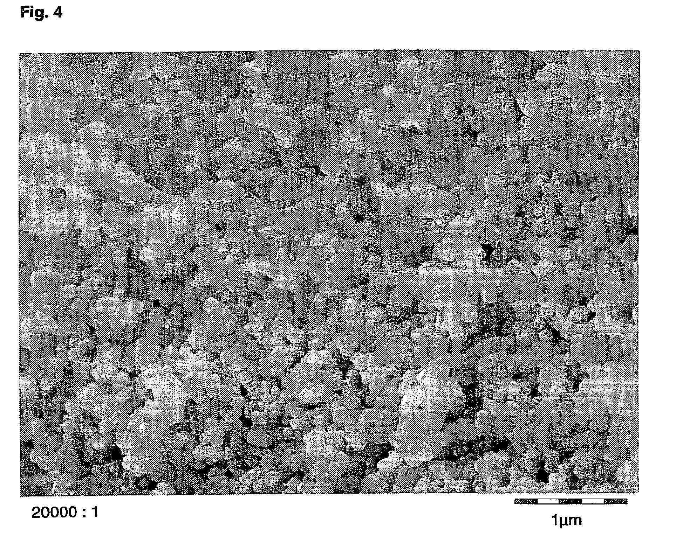

- Fig. 4: zeigt mit einer Auflösung von 20.000 : 1 eine SEM-Aufnahme des gemäß Beispiel 7 erhaltenen ZSM 5-Pulvers.

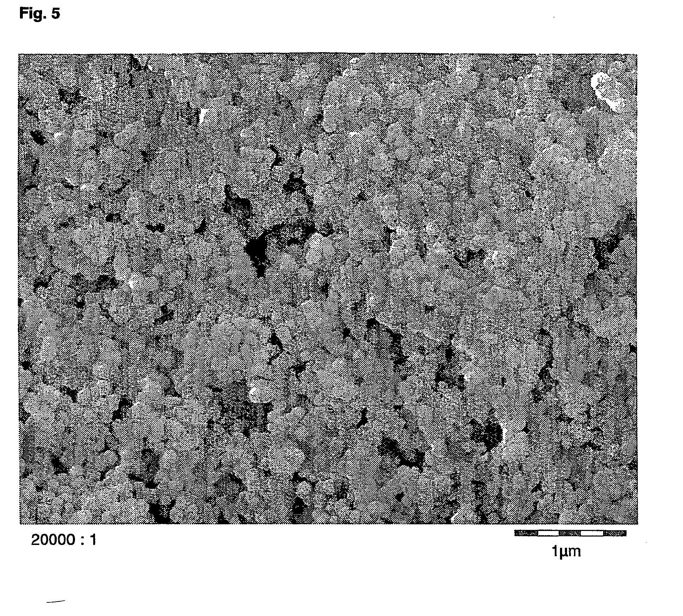

- Fig. 5: zeigt mit einer Auflösung von 20.000 : 1 eine SEM-Aufnahme des gemäß Beispiel 10 erhaltenen ZSM 5-Pulvers.

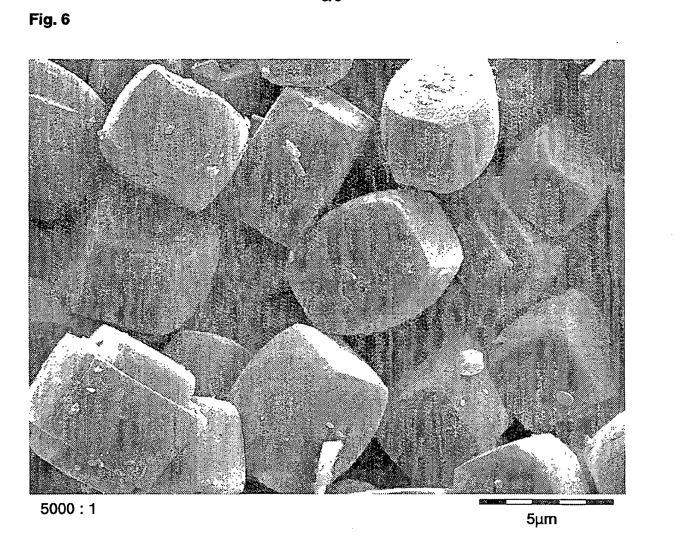

- Fig. 6: zeigt mit einer Auflösung von 5.000 : 1 eine SEM-Aufnahme des in Ver- gleichsbeispiel 4 verwendeten, kommerziell verfügbaren Zeolithpulvers PZ- 2/1000H (Fa. Zeochem, H-ZSM-5, molares Si : Al-Verhältnis von 500, abge- rundete quaderförmige Primärpartikel mit maximalen Kantenlängen a, b und c von 5, 4 und 2 µm und einem Na₂O-Gehalt von weniger als 100 mg/kg).

### Beispiele

### Beispiel 1: Herstellung von ZSM-5-Pulver

In einem Vierhalskolben wurden 920 g TEOS (Tetraethoxysilan) und 2,94 g Al₂(SO₄)₃•18H₂O vorgelegt und unter Rühren 1620 g einer 20 %-igen TPAOH (Tetrapropylammoniumhydroxid)-Lösung eingefüllt. Die Lösung wurde 10 min. bei Raumtemperatur gerührt. Danach wurde unter Normaldruck das durch Hydrolyse entstandene Ethanol abdestilliert, bis eine Sumpftemperatur von 95°C erreicht war. Es wurden 1485 g Synthesegel erhalten.

In einem 2,5L Stahlautoklaven wurden 742 g des Synthesegels zusammen mit 742 entionisiertem Wasser eingefüllt und das Gemisch unter Rühren 24 h bei 175°C unter Eigendruck gerührt. Nach Abkühlen auf Raumtemperatur wurde die gebildete Suspension mit konzentrierter HNO₃ auf einen pH-Wert von 7 titriert und die Suspension in einem Büchentrichter und über einem Papierfilter abfiltriert. Der Rückstand wurde zweimal mit je 500 ml Wasser gewaschen, danach im Trockenschrank bei Normaldruck 16 h bei 120 °C getrocknet und anschließend in einem Muffelofen mit Luftdosierung 5h bei 500°C kalziniert. Es wurden 137 g ZSM-5 Pulver erhalten.

Die Elektronenmikroskop-Aufnahme des ZSM-5-Pulvers bei 2x10⁴-facher Vergrößerung zeigte kugelförmige Primärpartikel mit einem Durchmesser jeweils zwischen 0,10 und 0,15 µm. Der Anteil der kugelförmigen Primärpartikel lag bei über 97 %. Der Gehalt an Na₂O betrug weniger als 50 mg/kg. Das molare Si : Al-Verhältnis betrug 460 : 1. Die BET-Oberfläche, bestimmt gemäß DIN 66131, lag bei 461 m²/g und das Porenvolumen, bestimmt gemäß DIN 66134, bei 1,10 cm³/g

### Beispiel 2: Herstellung eines Formkörpers, enthaltend ZSM-5-Zeolith

134 g ZSM-5 Pulver aus Beispiel 1 wurden zusammen mit 8,3 g Methylcellulose und 84 g Ludox AS40 (Fa. DuPont) in einem Kneter mechanisch gemischt. Danach wurden 110 ml entionisiertes Wasser zugegeben, die Masse 60 min. verdichtet und anschließend in einer Strangpresse mit 45 bar Druck zu 2 mm-Strängen verformt. Die Stränge wurden in einem Trockenschrank bei Normaldruck 16 h bei 120 °C getrocknet und anschließend in einem Muffelofen mit Luftdosierung 5 h bei 500 °C kalziniert. Es wurden 138 g ZSM-5 Katalysatorstränge mit einer Schneidhärte von 2,1 N erhalten. Die BET-Oberfläche, bestimmt gemäß DIN 66131, betrug 353 m²/g und das Porenvolumen, bestimmt gemäß DIN 66134, 0,62 cm³/g.

### Beispiel 3: Herstellung von Tetraethylendiamin (TEDA) aus Piperazin (PIP) und Ethylendiamin (EDA)

In einem sekundär mit Öl beheizten Rohrreaktor (Länge: 100 cm, Innendurchmesser: 6 mm), gefüllt mit 20 ml (= 11,4 g) Katalysator aus Beispiel 2, wurde ein Gemisch aus Ethylendiamin (EDA), Piperazin (PIP) und Wasser in einem Verhältnis von 25/25/50 Gew.-% bei einer Temperatur von 350°C und einer Belastung, bezogen auf die beiden organischen Komponenten EDA und PIP, von 0,50 g (organische Komponenten) / g (Katalysator) / h gefahren. Nach einer Laufzeit von 75 h wurde der Reaktionsaustrag über eine Periode von 1 h gesammelt und anschließend gaschromatographisch untersucht. Die Analyse des Reaktionsaustrages ergab einen Umsatz an EDA von 97 %, einen Umsatz an PIP von 48 % und eine Selektivität an TEDA von 95 %.

### Beispiel 4: Herstellung von ZSM-5-Pulver

Die Herstellung erfolgte analog zu Beispiel 1, ausgehend von 920 g TEOS, 2,94 g Al₂(SO₄)₃•18H₂O und 810 g einer 20%-igen TPAOH-Lösung. Die Ausbeute betrug 133 g.

Die Elektronenmikroskop-Aufnahme bei 2x10⁴-facher Vergrößerung zeigte kugelförmige ZSM-5-Primärpartikel. Der Anteil der kugelförmigen Primärpartikel lag bei über 97 %. Der Durchmesser der kugelförmigen Primärpartikeln lag zwischen 0,20 und 0,25 µm. Für die kugelförmigen Primärpartikel, die Quader mit abgerundeten Kanten darstellten, betrugen die maximalen Kantenlängen a, b und c, 0,2, 0,2 und 0,15 µm. Der Gehalt an Na₂O betrug weniger als 50 mg/kg. Das molare Si : Al-Verhältnis betrug 470 : 1. Die BET-Oberfläche, bestimmt gemäß DIN 66131, lag bei 440 m²/g und das Porenvolumen, bestimmt gemäß DIN 66134, bei 0,98 cm³/g

### Beispiel 5: Herstellung eines Formkörpers, enthaltend ZSM-5-Zeolith

Die Verformung erfolgte analog zu Beispiel 2, ausgehend von 126 g des ZSM-5 Pulvers aus Beispiel 4. Man erhielt 147 g Katalysatorstränge mit einer Schneidhärte von 2,9 N. Die BET-Oberfläche, bestimmt gemäß DIN 66131, betrug 382 m²/g und das Porenvolumen, bestimmt gemäß DIN 66134, 0,64 cm³/g.

### Beispiel 6: Herstellung von Tetraethylendiamin (TEDA) aus Piperazin (PIP) und Ethylendiamin (EDA)

Der Katalysator aus Beispiel 5 wurde analog zu Beispiel 3 getestet. Die Analyse des Reaktionsaustrages nach einer Laufzeit von 43 h ergab einen Umsatz an EDA von 95 %, einen Umsatz an PIP von 43 % und eine Selektivität an TEDA von 95 %.

### Beispiel 7: Herstellung von ZSM-5-Pulver

In einem Vierhalskolben wurden 460 g TEOS vorgelegt und unter Rühren 810 g einer 20 %igen TPAOH-Lösung eingefüllt. Die Lösung wurde 10 min. bei Raumtemperatur gerührt. Danach wurde unter Normaldruck das durch Hydrolyse entstandene Ethanol abdestilliert, bis eine Sumpftemperatur von 95°C erreicht war. Man erhielt 698 g Synthesegel ohne Aluminium.

In einem 2,5L Stahlautoklaven wurde das Synthesegel zusammen mit 698 ml entionisiertem Wasser und 1,84 g Al₂(SO₄)₃•18H₂O eingebaut. Die Reaktionsbedingungen und die Aufarbeitung des Reaktionsgemisches entsprachen denen des Beispiels 1. Man erhielt 132 g ZSM-5 Pulver.

Die Elektronenmikroskop-Aufnahme bei 2x10⁴-facher Vergrößerung zeigte kugelförmige ZSM-5-Primärpartikel mit einem Durchmesser zwischen 0,10 und 0,15 µm. Der Anteil der kugelförmigen Primärpartikel lag bei über 97 %. Der Anteil der kugelförmigen Primärpartikel lag bei mehr als 97 Gew.-%. Der Gehalt an Na₂O betrugt < 50 mg/kg. Das molare Si : AL-Verhältnis betrugt 385 : 1. Die BET-Oberfläche, bestimmt gemäß DIN 66131, beträgt 458 m²/g und das Porenvolumen, bestimmt gemäß DIN 66134, 1,24 cm³/g.

### Beispiel 8: Herstellung eines Formkörpers, enthaltend ZSM-5-Zeolith

Die Verformung erfolgte analog zu Beispiel 2, ausgehend von 128 g des ZSM-5 Pulvers aus Beispiel 7. Man erhielt 148 g ZSM-5 Katalysatorstränge mit einer Schneidhärte von 3,2 N. Die BET-Oberfläche, bestimmt gemäß DIN 66131, betrug 364 m²/g und das Porenvolumen, bestimmt gemäß DIN 66134, 0,68 cm³/g.

### Beispiel 9: Herstellung von Tetraethylendiamin (TEDA) aus Piperazin (PIP) und Ethylendiamin (EDA)

Der Katalysator aus Beispiel 8 wurde analog zu Beispiel 3 getestet. Die Analyse des Reaktionsaustrages nach einer Laufzeit von 48 h ergab einen Umsatz an EDA von 98 %, einen Umsatz an PIP von 46 % und eine Selektivität an TEDA von 94 %.

### Beispiel 10: Behandlung eines ZSM-5-Pulvers

132 g ZSM-5 Pulver aus Beispiel 1 wurden zusammen mit 1300 g entionisiertem Wasser in einem 2,5L Stahlautoklaven 24 h bei 175 °C und Eigendruck gerührt. Nach Abkühlen auf Raumtemperatur wurde die Suspension in einem Büchentrichter und über einem Papierfilter abfiltriert. Der Rückstand wurde einmal mit 500 ml Wasser gewaschen, danach im Trockenschrank bei Normaldruck 16 h bei 120 °C getrocknet und anschließend in einem Muffelofen mit Luftdosierung 5 h bei 500 °C kalziniert. Man erhielt 121 g behandeltes ZSM-5 Pulver.

Die Elektronenmikroskop-Aufnahme bei 2x10⁴-facher Vergrößerung zeigte kugelförmige ZSM-5-Primärpartikel mit einem Durchmesser zwischen 0,10 und 0,15 µm. Der Gehalt an Na₂O betrug weniger als 150 mg/kg. Das molare Si : Al-Verhältnis betrug 459 : 1. Die BET-Oberfläche, bestimmt gemäß DIN 66131, betrug 456 m²/g und das Porenvolumen, bestimmt gemäß DIN 66134, 1,12 cm³/g.

### Beispiel 11: Herstellung eines Formkörpers, enthaltend behandelten ZSM-5-Zeolith

Die Verformung erfolgte analog zu Beispiel 2, ausgehend von 121 g des ZSM-5 Pulvers aus Beispiel 10. Man erhielt 137 g ZSM-5 Katalysatorstränge mit einer Schneidhärte von 3,4 N. Die BET-Oberfläche, bestimmt gemäß DIN 66131, betrug 327 m²/g und das Porenvolumen, bestimmt gemäß DIN 66134, 0,69 cm³/g.

### Beispiele 12: Herstellung von Tetraethylendiamin (TEDA) aus Piperazin (PIP) und Ethylendiamin (EDA)

Der Katalysator aus Beispiel 11 wurde analog zu Beispiel 3 getestet. Die Analyse des Reaktionsaustrages nach einer Laufzeit von 47 h ergab einen Umsatz an EDA von 99 %, einen Umsatz an PIP von 49 % und eine Selektivität an TEDA von 96 %

### Beispiel 13: Herstellung von Tetraethylendiamin (TEDA) aus Piperazin (PIP) und Ethylendiamin (EDA)

Die Versuchsdurchführung erfolgte analog zu Beispiel 3 mit dem Katalysator aus Beispiel 11, ausgehend von einem Gemisch aus Ethylendiamin (EDA), Piperazin (PIP) und Wasser in einem Verhältnis von 40/10/50 Gew.-%, bei einer Temperatur von 350 °C und einer Belastung, bezogen auf die beiden organischen Komponenten EDA und PIP von 0,30 g (organische Komponenten) / g (Katalysator) / h. Die Analysen der Reaktionsausträge nach einer Laufzeit von 162, 716 und 1147 h sind in der Tabelle 1 zusammengefasst.

**Tabelle 1**

| Laufzeit / h | Umsatz an EDA / % | Selektivität an TEDA / % |
|---|---|---|
| 162 | 98 | 93 |
| 716 | 97 | 93 |
| 1147 | 96 | 93 |

Durch die Wasserbehandlung des ZSM-5-Pulvers unter hydrothermalen Bedingungen wurde demgemäß sowohl die Aktivität des Katalysators als auch die Selektivität verbessert. Unter PIP-neutraler Fahrweise konnte bei gleichbleibender TEDA-Selektivität und einem EDA-Umsatz von mehr als 95% eine Laufzeit von mindestens 1147 h erreicht werden.

### Vergleichsbeispiel 1: Herstellung von ZSM-5-Pulver unter Zugabe von NaOH

Die Herstellung des ZSM-5-Pulvers erfolgte analog zu Beispiel 4 mit zusätzlich 0,80 g NaOH. Die Ausbeute betrug 135 g.

Die Elektronenmikroskop-Aufnahme bei 2x10⁴-facher Vergrößerung zeigte kugelförmige ZSM-5-Primärpartikel mit einem Durchmesser zwischen 0,10 und 0,15 µm. Der Gehalt an Na₂O betrug 853 mg/kg. Das molare Si : Al-Verhältnis beträgt 475 : 1. Die BET-Oberfläche, bestimmt gemäß DIN 66131, betrug 464 m²/g und das Porenvolumen, bestimmt gemäß DIN 66134, 0,95 cm³/g.

### Vergleichsbeispiel 2: Herstellung eines Formkörpers, enthaltend behandelten ZSM-5-Zeolith

Die Verformung erfolgte analog zu Beispiel 2, ausgehend von 120 g ZSM-5 Pulver aus Vergleichsbeispiel 1. Man erhielt 131 g ZSM-5 Katalysatorstränge mit einer Schneidhärte von 6,1 N. Die BET-Oberfläche, bestimmt gemäß DIN 66131, betrug 332 m²/g und das Porenvolumen, bestimmt gemäß DIN 66134, 0,63 cm³/g.

### Vergleichsbeispiel 3: Herstellung von Tetraethylendiamin (TEDA) aus Piperazin (PIP) und Ethylendiamin (EDA)

Der Katalysator aus Vergleichsbeispiel 2 wurde analog zu Beispiel 3 getestet. Die Analyse des Reaktionsaustrages nach einer Laufzeit von 30 h ergab einen Umsatz an EDA von 92 %, einen Umsatz an PIP von 45 % und eine Selektivität an TEDA von 94 %. Die erhöhte Na-Konzentration führte somit zu einer Senkung des EDA-Umsatzes.

### Vergleichsbeispiel 4: Herstellung eines Formkörpers, enthaltend kommerziellen ZSM-5-Zeolith

120 g des kommerziell verfügbaren Zeolithpulvers PZ-2/1000H (Fa. Zeochem, H-ZSM-5, molares Si : Al-Verhältnis von 500, abgerundete quaderförmige Primärpartikel mit maximalen Kantenlängen a, b und c von 5, 4 und 2 µm und einem Na₂O-Gehalt von weniger als 100 mg/kg) wurden analog zu Beispiel 2 mit Ludox AS40 verformt. Man erhielt 133 g ZSM-5 Katalysatorstränge mit einer Schneidhärte von 3,9 N. Die BET-Oberfläche, bestimmt gemäß DIN 66131, betrug 337 m²/g und das Porenvolumen, bestimmt gemäß DIN 66134, 0,23 cm³/g.

### Vergleichsbeispiel 5: Herstellung von Tetraethylendiamin (TEDA) aus Piperazin (PIP) und Ethylendiamin (EDA)

Der Katalysator aus Vergleichsbeispiel 4 wurde analog zu Beispiel 3 getestet. Die Analyse des Reaktionsaustrages nach einer Laufzeit von 46 h ergab einen Umsatz an EDA von 98 %, einen Umsatz an PIP von 42 % und eine Selektivität an TEDA von 88 %.

Die Verwendung von ZSM-5 mit einer Partikelgröße von mehr als 1 µm führte zu einer deutlich niedrigeren TEDA-Selektivität.

## Patentansprüche

1. Zeolithisches Material vom Pentasiltyp mit einem Alkali- und Erdalkaligehalt von höchstens 150 ppm und einem molaren Verhältnis von Si zu Al im Bereich von 250 bis 1500, wobei mindestens 90 % der Primärpartikel des zeolithischen Materials kugelförmig sind und mindestens 95 Gew.-% der kugelförmigen Primärpartikel einen Durchmesser im Bereich von kleiner oder gleich 1 µm aufweisen.

2. Zeolithisches Material nach Anspruch 1, **dadurch gekennzeichnet, dass** es zumindest teilweise den Strukturtyp ZSM-5 aufweist.

3. Zeolithisches Material nach Anspruch 1 oder 2, **dadurch gekennzeichnet, dass** der Alkali- und Erdalkaligehalt des zeolithischen Materials höchstens 100 ppm beträgt.

4. Zeolithisches Material nach einem der Ansprüche 1 bis 3, **dadurch gekennzeichnet, dass** der Durchmesser der kugelförmigen Primärpartikel im Bereich von 50 bis 250 nm liegt.

5. Zeolithisches Material nach einem der Ansprüche 1 bis 4, **dadurch gekennzeichnet, dass** das molare Verhältnis von Si zu Al im Bereich von 250 bis 750 liegt.

6. Zeolithisches Material nach einem der Ansprüche 1 bis 5, **dadurch gekennzeichnet, dass** das molare Verhältnis von Si zu Al im Bereich von 350 bis 600 liegt.

7. Formkörper, enthaltend mindestens ein zeolithisches Material gemäß einem der Ansprüche 1 bis 6.

8. Formkörper nach Anspruch 7, zusätzlich enthaltend SiO₂ als Bindermaterial.

9. Formkörper nach Anspruch 7 oder 8, enthaltend Bindermaterial in einem Bereich von 5 bis 80 Gew.-%, bezogen auf das Gesamtgewicht des getrockneten und gegebenenfalls calcinierten Formkörpers.

10. Formkörper nach einem der Ansprüche 7 bis 9 mit einer spezifischen Oberfläche von mindestens 350 m²/g, enthaltend Poren mit einem Porenvolumen von mindestens 0,6 ml/g.

11. Formkörper nach einem der Ansprüche 7 bis 10 mit einer Schneidhärte im Bereich von 2 bis 15 N.

12. Verfahren zur Herstellung eines zeolithischen Materials gemäß einem der Ansprüche 1 bis 6, umfassend die Schritte
(i) Herstellung eines Gemischs, enthaltend mindestens eine SiO₂-Quelle, mindestens eine Aluminiumquelle und mindestens eine Templatverbindung, wobei das Gemisch höchstens 150 ppm Alkali- und Erdalkalimetall enthält und wobei die mindestens eine SiO₂-Quelle und die mindestens eine Aluminiumquelle in einem Mengenverhältnis eingesetzt werden, die die Bildung eines kristallinen Materials mit einem molaren Verhältnis von Si zu Al im Bereich von 250 bis 1500 erlaubt,
(ii) Umsetzung der im Gemisch enthaltenden Verbindungen unter Erhalt einer Mutterlauge, enthaltend kristallines, mindestens einen Teil mindestens einer Templatverbindung enthaltendes Material;
(iii) Abtrennen des kristallinen Materials aus der Mutterlauge;
(iv) Entfernen der mindestens einen Templatverbindung aus dem kristallinen Material.

13. Verfahren nach Anspruch 12, wobei als SiO₂-Quelle ein Tetraalkoxysilan und als Templatverbindung mindestens ein Tetraalkylammoniumhydroxid eingesetzt werden und das Gemisch gemäß (i) zusätzlich Wasser enthält.

14. Verfahren nach Anspruch 13, wobei vor der Umsetzung gemäß (ii) der im Gemisch gemäß (i) entstehende Alkohol abdestilliert wird.

15. Verfahren nach einem der Ansprüche 12 bis 14, wobei die Umsetzung gemäß (ii) bei einer Temperatur im Bereich von 150 bis 180 °C in einem Autoklaven bei einer Umsetzungsdauer von 1 bis 48 h umgesetzt wird.

16. Verfahren nach einem der Ansprüche 12 bis 15, wobei das gemäß (iii) abgetrennte kristalline Material gemäß (iv) zunächst bei einer Temperatur im Bereich von 100 bis 160°C getrocknet und anschließend bei einer Temperatur im Bereich von 450 bis 700 °C calciniert wird.

17. Verfahren nach einem der Ansprüche 12 bis 16, wobei nach Schritt (iv) das zeolithische Material in einem Autoklaven mit Wasser in Kontakt gebracht wird und anschließend bei einer Temperatur im Bereich von 80 bis 160°C getrocknet und anschließend bei einer Temperatur im Bereich von 400 bis 750 °C calciniert wird.

18. Verfahren zur Herstellung eines Formkörpers gemäß einem der Ansprüche 7 bis 11, umfassend die Schritte
(I) Herstellen eines Gemischs, enthaltend ein zeolithisches Material gemäß einem der Ansprüche 1 bis 6 oder ein zeolithisches Material, erhältlich durch ein Verfahren gemäß einem der Ansprüche 12 bis 17, und mindestens ein Bindermaterial;
(II) Kneten des Gemischs;
(III) Verformen des gekneteten Gemischs unter Erhalt mindestens eines Formkörpers;
(IV) Trocknen des mindestens einen Formkörpers;
(V) Calcinieren des getrockneten Formkörpers.

19. Verfahren nach Anspruch 18, wobei das gemäß (I) eingesetzte Bindermaterial ein SiO₂ enthaltendes Bindermaterial ist.

20. Verfahren nach Anspruch 18 oder 19, wobei das Gemisch gemäß (I) zusätzlich mindestens einen Porenbildner enthält.

21. Formkörper, erhältlich durch ein Verfahren gemäß einem der Ansprüche 18 bis 20.

22. Verfahren zur Herstellung von Triethylendiamin oder eines alkylsubstituierten Derivats davon durch Umsetzung mindestens eines Eduktes, das eine Struktureinheit gemäß Formel (I) 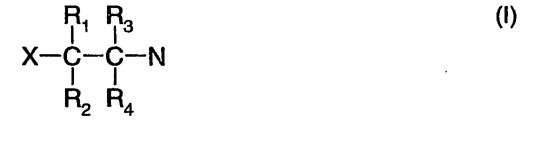 aufweist, wobei R₁, R₂, R₃ und R₄ unabhängig voneinander ein Wasserstoffatom oder eine Alkylgruppe mit 1 bis 4 Kohlenstoffatomen und X ein Sauerstoff- oder Stickstoffatom darstellen, **dadurch gekennzeichnet, dass** die Umsetzung an einem Zeolithkatalysator erfolgt, der ein zeolithisches Material gemäß einem der Ansprüche 1 bis 6 oder ein zeolithisches Material, erhältlich durch ein Verfahren gemäß einem der Ansprüche 12 bis 17, enthält.

23. Verfahren nach Anspruch 22 durch Umsetzung eines Eduktes, bestehend aus
(A) x Gew.-% Piperazin (PIP= und
(B) y Gew.% Ethylendiamin (EDA),
wobei x + y = 1 00 und 0 ≤ x ≤ 100 und 0 ≤ y ≤ 100.

24. Verfahren nach Anspruch 23, **dadurch gekennzeichnet, dass** das Edukt in mindestens einem Lösungs- oder Verdünnungsmittel umgesetzt wird.

25. Verfahren nach einem der Ansprüche 22 bis 24, wobei mindestens ein Teil des zeolithischen Materials des Katalysators in der H-Form eingesetzt wird.

26. Verfahren nach einem der Ansprüche 23 bis 25, wobei x gleich 0 ist und die Umsetzung bei einer Temperatur im Bereich von 300 bis 400 °C und einem Druck im Bereich von 0,01 bis 50 bar durchgeführt wird.

27. Verfahren nach einem der Ansprüche 23 bis 25, wobei y gleich 0 ist und die Umsetzung bei einer Temperatur im Bereich von 300 bis 450°C und einem Druck im Bereich von 0,01 bis 50 bar durchgeführt wird.

28. Verfahren nach einem der Ansprüche 23 bis 25, wobei x und y ungleich 0 sind und Wasser, EDA und PIP zu 10 bis 50 Gew.-% bezüglich Wasser und zu 90 bis 50 Gew.-% bezüglich der Summe der Gewichte von PIP und EDA vorliegen.

29. Verfahren nach Anspruch 28, wobei die Umsetzung bei einer Temperatur im Bereich von 290 bis 400 °C und einem Druck im Bereich von 0,01 bis 10 bar durchgeführt wird.

30. Verfahren nach Anspruch 28 oder 29, wobei EDA und PIP im Gewichtsverhältnis im Bereich von 1:1 bis 10:1, gerechnet als Verhältnis von Gewicht EDA zu Gewicht PIP, vorliegen.

## Claims

1. A zeolite material of the pentasil type having an alkali metal and alkaline earth metal content of not more than 150 ppm and a molar ratio of Si to Al of from 250 to 1500, at least 90% of the primary particles of the zeolite material being spherical and at least 95% by weight of the spherical primary particles having a diameter of less than or equal to 1 µm.

2. The zeolite material according to claim 1, which at least partly has the structure type ZSM-5.

3. The zeolite material according to claim 1 or 2, wherein the alkali metal and alkaline earth metal content of the zeolite material is not more than 100 ppm.

4. The zeolite material according to any of claims 1 to 3, wherein the diameter of the spherical primary particles is from 50 to 250 nm.

5. The zeolite material according to any of claims 1 to 4, wherein the molar ratio of Si to Al is from 250 to 750.

6. The zeolite material according to any of claims 1 to 5, wherein the molar ratio of Si to Al is from 350 to 600.

7. A molding comprising at least one zeolite material according to any of claims 1 to 6.

8. The molding according to claim 7, additionally comprising SiO₂ as binder material.

9. The molding according to claim 7 or 8, comprising binder material in a range of from 5 to 80% by weight, based on the total weight of the dried and, if required, calcined molding.

10. The molding according to any of claims 7 to 9, having a specific surface area of at least 350 m²/g, comprising pores having a pore volume of at least 0.6 ml/g.

11. The molding according to any of claims 7 to 10, having a cutting hardness of from 2 to 15 N.

12. A process for the preparation of a zeolite material according to any of claims 1 to 6, comprising the steps
(i) preparation of a mixture comprising at least one SiO₂ source, at least one aluminum source and at least one template compound, the mixture comprising not more than 150 ppm of alkali metal and alkaline earth metal and the at least one SiO₂ source and the at least one aluminum source being used in a ratio which permits the formation of a crystalline material having a molar ratio of Si to Al of from 250 to 1500;
(ii) reaction of the compounds present in the mixture to give a mother liquor comprising crystalline material comprising at least a part of the at least one template compound;
(iii) isolation of the crystalline material from the mother liquor;
(iv) removal of the at least one template compound from the crystalline material.

13. The process according to claim 12, a tetraalkoxysilane being used as the SiO₂ source and at least one tetraalkylammonium hydroxide as the template compound, and the mixture according to (i) additionally comprising water.

14. The process according to claim 13, the alcohol forming in the mixture according to (i) being distilled off before the reaction according to (ii).

15. The process according to any of claims 12 to 14, the reaction according to (ii) being carried out at from 150 to 180°C in an autoclave for a reaction time of 1 to 48 hours.

16. The process according to any of claims 12 to 15, the crystalline material according to (iv) which is isolated according to (iii) first being dried at from 100 to 160°C and then being calcined at from 450 to 700°C.

17. The process according to any of claims 12 to 16, the zeolite material, after step (iv), being brought into contact with water in an autoclave and then being dried at from 80 to 160°C and then being calcined at from 400 to 750°C.

18. The process for the production of a molding according to any of claims 7 to 11, comprising the steps
(I) preparation of a mixture comprising a zeolite material according to any of claims 1 to 6, or a zeolite material obtainable by a process according to any of claims 12 to 17, and at least one binder material;
(II) kneading of the mixture;
(III) molding of the kneaded mixture to give at least one molding;
(IV) drying of the at least one molding;
(V) calcination of the dried molding.

19. The process according to claim 18, the binder material used according to (I) being an SiO₂-comprising binder material.

20. The process according to claim 18 or 19, the mixture according to (I) additionally comprising at least one pore former.

21. A molding obtainable by a process according to any of claims 18 to 20.

22. A process for the preparation of triethylenediamine or of an alkyl-substituted derivative thereof by reacting at least one starting material which has a structural unit according to formula (I) 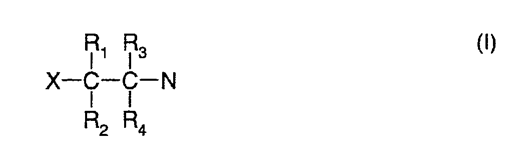 where R₁, R₂, R₃ and R₄, independently of one another, are hydrogen or alkyl of 1 to 4 carbon atoms and X is oxygen or nitrogen, wherein the reaction is carried out over a zeolite catalyst which comprises a zeolite material according to any of claims 1 to 6 or a zeolite material obtainable by a process according to any of claims 12 to 17.

23. The process according to claim 22, which comprises reacting a starting material consisting of
(A) x% by weight of piperazine (PIP) and
(B) y% by weight of ethylenediamine (EDA),
where x + y = 100 and 0 ≤ x ≤ 100 and 0 ≤ y ≤ 100.

24. The process according to claim 23, wherein the starting material is reacted in at least one solvent or diluent.

25. The process according to any of claims 22 to 24, at least a part of the zeolite material of the catalyst being used in the H form.

26. The process according to any of claims 23 to 25, x being 0 and the reaction being carried out at from 300 to 400°C and from 0.01 to 50 bar.

27. The process according to any of claims 23 to 25, y being 0 and the reaction being carried out at from 300 to 450°C and from 0.01 to 50 bar.

28. The process according to any of claims 23 to 25, x and y not being 0 and water, EDA and PIP being present to an extent of from 10 to 50% by weight based on water and 90 to 50% by weight based on the sum of the weights of PIP and EDA.

29. The process according to claim 28, the reaction being carried out at from 290 to 400°C and from 0.01 to 10 bar.

30. The process according to claim 28 or 29, EDA and PIP being present in a weight ratio from 1:1 to 10:1, calculated as the ratio of the weight of EDA to the weight of PIP.

## Revendications

1. Matériau zéolithique du type Pentasil ayant une teneur en alcalins et alcalino-terreux d'au maximum 150 ppm et un rapport molaire de Si à Al dans la plage de 250 à 1 500, au moins 90 % des particules primaires du matériau zéolithique étant sphériques et au moins 95 % en poids des particules primaires sphériques ayant un diamètre dans la plage inférieure ou égale à 1 µm.

2. Matériau zéolithique selon la revendication 1, **caractérisé en ce qu'**il présente au moins partiellement le type de structure ZSM-5.

3. Matériau zéolithique selon la revendication 1 ou 2, **caractérisé en ce que** la teneur en alcalins et alcalino-terreux du matériau zéolithique est d'au maximum 100 ppm.

4. Matériau zéolithique selon l'une quelconque des revendications 1 à 3, **caractérisé en ce que** le diamètre des particules primaires sphériques se situe dans la plage de 50 à 250 nm.

5. Matériau zéolithique selon l'une quelconque des revendications 1 à 4, **caractérisé en ce que** le rapport molaire de Si à Al se situe dans la plage allant de 250 à 750.

6. Matériau zéolithique selon l'une quelconque des revendications 1 à 5, **caractérisé en ce que** le rapport molaire de Si à Al se situe dans la plage allant de 350 à 600.

7. Corps moulé, contenant au moins un matériau zéolithique selon l'une quelconque des revendications 1 à 6.

8. Corps moulé selon la revendication 7, contenant en outre du SiO₂ en tant que liant.

9. Corps moulé selon la revendication 7 ou 8, contenant un liant dans une plage de 5 à 80 % en poids, par rapport au poids total du corps moulé séché et éventuellement calciné.

10. Corps moulé selon l'une quelconque des revendications 7 à 9, ayant une surface spécifique d'au moins 350 m²/g, comportant des pores ayant un volume de pore d'au moins 0,6 ml/g.

11. Corps moulé selon l'une quelconque des revendications 7 à 10, ayant une dureté à la coupe dans la plage de 2 à 15 N.

12. Procédé pour la production d'un matériau zéolithique selon l'une quelconque des revendications 1 à 6, comprenant les étapes suivantes
(i) préparation d'un mélange contenant au moins une source de SiO₂, au moins une source d'aluminium et au moins un composé matrice, le mélange contenant au maximum 150 ppm de métal alcalin et alcalino-terreux et ladite au moins une source de SiO₂ et ladite au moins une source d'aluminium étant utilisées en un rapport de quantités permettant la formation d'un matériau cristallin ayant un rapport molaire de Si à Al dans la plage de 250 à 1 500,
(ii) mise en réaction des composés contenus dans le mélange, avec obtention d'une liqueur-mère contenant une substance cristalline contenant au moins une partie dudit au moins un composé matrice ;
(iii) séparation de la substance cristalline d'avec la liqueur-mère ;
(iv) séparation dudit au moins un composé matrice d'avec la substance cristalline.

13. Procédé selon la revendication 12, dans lequel on utilise comme source de SiO₂ un tétraalcoxysilane et en tant que composé matrice au moins un hydroxyde de tétraalkylammonium et le mélange selon (i) contient en outre de l'eau.

14. Procédé selon la revendication 13, dans lequel, avant la réaction selon (ii) on élimine par distillation l'alcool formé dans le mélange selon (i).

15. Procédé selon l'une quelconque des revendications 12 à 14, dans lequel la réaction selon (ii) est effectuée à une température dans la plage de 150 à 180 °C dans un autoclave pendant une durée de réaction de 1 à 48 heures.

16. Procédé selon l'une quelconque des revendications 12 à 15, dans lequel la substance cristalline séparée selon (iii) est, conformément à (iv), d'abord séchée à une température dans la plage de 100 à 160 °C, et ensuite calcinée à une température dans la plage de 450 à 700 °C.

17. Procédé selon l'une quelconque des revendications 12 à 16, dans lequel après l'étape (iv) le matériau zéolithique est mis en contact avec de l'eau dans un autoclave et ensuite séché à une température dans la plage de 80 à 160 °C et ensuite calciné à une température dans la plage de 400 à 750 °C.

18. Procédé pour la production d'un corps moulé selon l'une quelconque des revendications 7 à 11, comprenant les étapes suivantes
(I) préparation d'un mélange contenant un matériau zéolithique selon l'une quelconque des revendications 1 à 6, ou un matériau zéolithique pouvant être obtenu par un procédé selon l'une quelconque des revendications 12 à 17, et au moins un liant ;
(II) malaxage du mélange ;
(III) mise en forme du mélange malaxé, avec obtention d'au moins un corps moulé ;
(IV) séchage dudit au moins un corps moulé ;
(V) calcination du corps moule sec.

19. Procédé selon la revendication 18, dans lequel le liant utilisé selon (I) est un liant contenant SiO₂.

20. Procédé selon la revendication 18 ou 19, dans lequel le mélange selon (I) contient en outre au moins un agent porogène.

21. Corps moulé, pouvant être obtenu par un procédé selon l'une quelconque des revendications 18 à 20.

22. Procédé pour la préparation de triéthylènediamine ou d'un dérivé de celle-ci substitué par alkyle, par mise en réaction d'au moins un produit de départ qui présente un motif structural selon la formule (I) 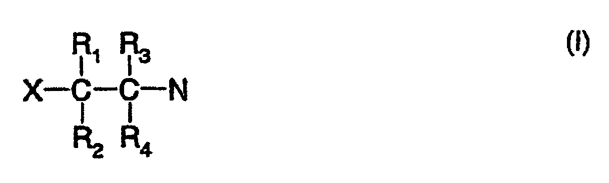 dans laquelle R₁, R₂, R₃ et R₄ représentent, indépendamment les uns des autres, un atome d'hydrogène ou un groupe alkyle ayant de 1 à 4 atomes de carbone et X représente un atome d'oxygène ou d'azote, **caractérisé en ce que** la réaction s'effectue sur un catalyseur zéolithique qui contient un matériau zéolithique selon l'une quelconque des revendications 1 à 6 ou un matériau zéolithique pouvant être obtenu selon l'une quelconque des revendications 12 à 17.

23. Procédé selon la revendication 22, **caractérisé par** mise en réaction d'un produit de départ constitué de
(A) x % en poids de pipérazine (PIP) et
(B) y % en poids d'éthylènediamine (EDA),
la somme x + y étant égale à 100 et 0 ≤ x ≤ 100 et 0 ≤ y ≤ 100.

24. Procédé selon la revendication 23, **caractérisé en ce que** le produit de départ est mis en réaction dans au moins un solvant ou diluant.

25. Procédé selon l'une quelconque des revendications 22 à 24, dans lequel au moins une partie du matériau zéolithique du catalyseur est utilisée sous la forme H.

26. Procédé selon l'une quelconque des revendications 23 à 25, dans lequel x est égal à 0 et la réaction est effectuée à une température dans la plage de 300 à 400 °C et sous une pression dans la plage de 0,01 à 50 bars.

27. Procédé selon l'une quelconque des revendications 23 à 25, dans lequel y est égal à 0 et la réaction est effectuée à une température dans la plage de 300 à 450 °C et sous une pression dans la plage de 0,01 à 50 bars.

28. Procédé selon l'une quelconque des revendications 23 à 25, dans lequel x et y sont différents de 0 et de l'eau, EDA et PIP sont présentes à raison de 10 à 50 % en poids en ce qui concerne l'eau et de 90 à 50 % en poids en ce qui concerne la somme des poids de PIP et EDA.

29. Procédé selon la revendication 28, dans lequel la réaction est effectuée à une température dans la plage de 290 à 400 °C et sous une pression dans la plage de 0,01 à 10 bars.

30. Procédé selon la revendication 28 ou 29, dans lequel EDA et PIP sont présentes en un rapport pondéral dans la plage de 1:1 à 10:1, calculé en relation du poids d'EDA au poids de PIP.
